# EUROPEAN PATENT SPECIFICATION

(11) **EP 3 951 495 B1**
(45) Date of publication and mention of the grant of the patent: **07.05.2025**
(21) Application number: 20784279.0
(22) Date of filing: 20.01.2020
(51) Int. Cl.: A61B 5/00, G03B 15/05, G03B 11/04, H04N 23/56

(54) **ILLUMINATION DEVICE AND IMAGING DEVICE**
BELEUCHTUNGSVORRICHTUNG UND ABBILDUNGSVORRICHTUNG
DISPOSITIF D'ÉCLAIRAGE ET DISPOSITIF D'IMAGERIE

(30) Priority: 29.03.2019 JP 2019066958; 24.05.2019 JP 2019098137
(43) Date of publication of application: 09.02.2022
(73) Proprietor: Casio Computer Co., Ltd., Tokyo 151-8543 (JP)
(72) Inventor: YOSHIDA, Tohru, Hamura-shi, Tokyo 205-8555 (JP); MATSUO, Katsuyuki, Hamura-shi, Tokyo 205-8555 (JP)
(74) Representative: Grünecker Patent- und Rechtsanwälte PartG mbB
(86) International application number: PCT/JP2020/001663
(87) International publication number: WO 2020/202710

(56) References cited:
- WO-A1-2015/093314
- WO-A1-2018/135337
- JP-A- 2015 018 857
- JP-A- 2018 005 155
- JP-A- 2018 010 007
- US-A1- 2011 184 260
- US-A1- 2016 366 316
- US-A1- 2018 279 942
- US-B1- 6 993 167

## Description

The present disclosure relates to an illumination device and an imaging device including the illumination device.

A known imaging device including an illumination device is described in, for example, JP 2012 - 205 855 A. The imaging device includes an objective glass to be in contact with human skin through a gel, multiple light sources that emit light, and an imager for imaging. The skin in contact with the objective glass is illuminated by the multiple light sources inside the imaging device and imaged by the imager.
US 2018 / 279 942 A1 discloses a medical imaging device that changes a state between a first capturing state for capturing a lesion in a regular capturing state and a second capturing state for capturing a lesion in a capturing state that is different from the regular capturing state and captures an image for assisting in diagnosis of the lesion. The medical imaging device includes an imaging device body that includes a first light source, a second light source, an imaging element, a set of lenses that are situated on an optical axis that connects the imaging element and an object, uses at least the first light source as a light source that emits light to the object in the first capturing state, and uses at least the second light source as a light source that emits light to the object in the second capturing state.
US 6 993 167 B1 describes a system for collecting, storing and displaying dermatological images for the purpose of monitoring and diagnosis of skin conditions and skin cancers, including melanoma. A hand-held unit illuminates a section of the patient's skin, and an imaging device generates imaging signals from light derived from a skin section. Pairs of light output ports in the hand-held unit are arranged such that their intensity distributions overlap at their half-intensity levels so that the resulting summation of their intensities has a flat central region. Three image stores are maintained, one for lesion images, one for "nearby skin" images, and one for reference-white images. The "nearby skin" images are used by the system software to automatically determine the skin/lesion border. The reference white images are used to set the dynamic range of the instrument and to compensate for lighting irregularities. Two images of the same lesion taken at different times may be displayed simultaneously so that changes in the lesion may be determined. The calibration system is designed so that image data taken on any of multiple machines built to the same specification will be corrected back to a common reference standard to ensure absolute accuracy in colour rendition.

A typical imaging device for imaging skin includes the objective glass to be in contact with the skin surface through a gel for preventing light reflection from the skin surface. However, light from the light sources illuminating the skin may be reflected by the objective glass depending on the positions of the light sources, possibly disabling proper imaging of the skin. Such reflection may also occur in imaging targets other than skin.

It is an object of the present invention to provide an illumination device that allows proper imaging of a target by reducing the likelihood of imaging being affected by reflected light of light sources and an imaging device including the illumination device. According to the present invention said object is solved by an imaging device having the features of the independent claim 1. Preferred embodiments are laid down in the dependent claims.

The technique according to the above aspect of the present disclosure allows proper imaging of a target by reducing the likelihood of imaging being affected by reflected light of the light source.

### Brief Description of Drawings

FIG. 1A is a perspective view of a dermatoscopy camera according to Embodiment 1 of the present disclosure, without an adapter being attached;
FIG. 1B is a perspective view of the dermatoscopy camera according to Embodiment 1 of the present disclosure, with the adapter attached;
FIG. 2 is an exploded perspective view of the dermatoscopy camera;
FIG. 3 is a perspective cross-sectional view of an imager included in the dermatoscopy camera;
FIG. 4 is a front view of an illumination device included in the dermatoscopy camera as viewed in the direction indicated by arrow IV in FIG. 1A;
FIG. 5 is a cross-sectional view of the illumination device taken along line V-V in FIG. 4;
FIG. 6 is a cross-sectional view of the illumination device with the adapter attached;
FIG. 7 is a front view of a base included in the body of the illumination device;
FIG. 8 is a front view of the base included in the body of the illumination device with a first light-emitting diode (LED) board removed from the base;
FIG. 9 is an enlarged view of portion IX in FIG. 5;
FIG. 10 is a partial cross-sectional view of the dermatoscopy camera;
FIG. 11 is a schematic diagram describing light illumination during second dermatoscopy imaging;
FIG. 12 is a partial cross-sectional view of the dermatoscopy camera with the adapter attached;
FIG. 13 is a schematic diagram describing light illumination during the second dermatoscopy imaging;
FIG. 14A is a diagram showing illuminance distribution on a second objective cover obtained through simulation in which LEDs for the second dermatoscopy imaging are turned on;
FIG. 14B is a diagram showing illuminance distribution on the second objective cover obtained through simulation in which LEDs for first dermatoscopy imaging are turned on;
FIG. 15 is a flowchart showing an imaging operation performed by the dermatoscopy camera;
FIG. 16 is a flowchart showing a dermatoscopy imaging operation;
FIG. 17 is a flowchart showing a first dermatoscopy imaging operation;
FIG. 18 is a flowchart showing a second dermatoscopy imaging operation;
FIG. 19 is a diagram describing the focus on a target that is set for imaging;
FIG. 20 is a partial cross-sectional view of a dermatoscopy camera according to Embodiment 2;
FIG. 21 is a partial cross-sectional view of the dermatoscopy camera according to Embodiment 2 with an adapter attached;
FIG. 22A is a diagram describing movement of LED boards in a dermatoscopy camera according to a modification of the present disclosure;
FIG. 22B is a diagram describing rotation of LED boards in a dermatoscopy camera according to a modification of the present disclosure not covered by the claims; and
FIG. 23 is a flowchart showing an imaging operation of a dermatoscopy camera according to a modification of the present disclosure not covered by the claims.

### Description of Embodiments

A dermatoscopy camera according to one or more embodiments of the present disclosure will now be described with reference to the drawings. The term dermatoscope herein refers to a magnifier (device) for skin examinations, and the term dermatoscopy refers to skin examinations using the magnifier or to the use of (the act of using) the magnifier, similarly to the use of the terms microscope and microscopy.

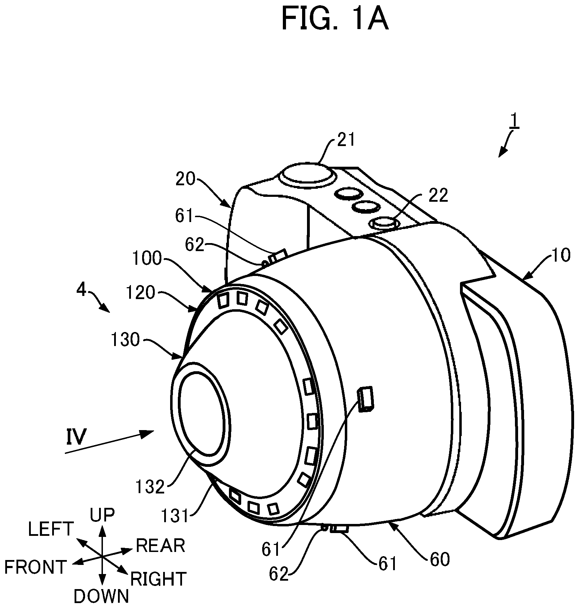

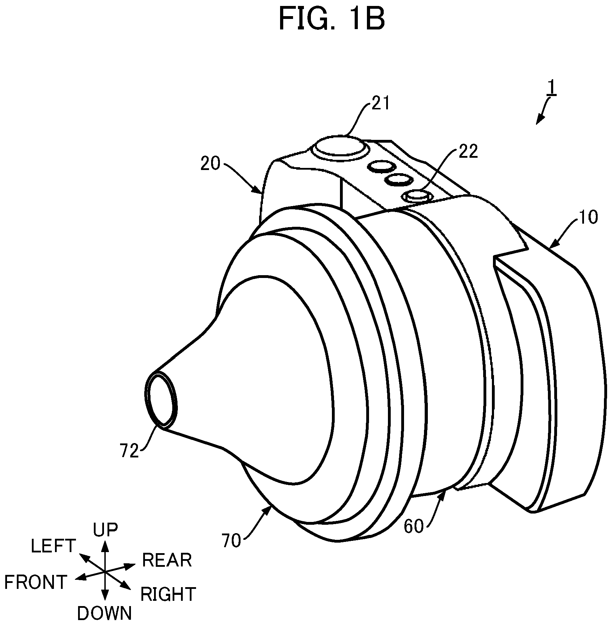

As in FIGS. 1A and 1B, the examples will be hereafter described based on an orthogonal coordinate system with the front (front surface) of a dermatoscopy camera 1 being nearer an imaging target (subject), the rear being opposite to the front, and the up-down (vertical) direction and the right-left (lateral) direction being the up-down (vertical) direction and the right-left (lateral) direction of the dermatoscopy camera 1 when viewed from the front. Unless otherwise specified, the components are fastened in any suitable manner, such as with threads or screws or by fitting.

### Embodiment 1

The dermatoscopy camera 1 according to Embodiment 1 is an example of an imaging device that captures an image for examination of skin conditions. The dermatoscopy camera 1 can image an imaging target either with an adapter 70 attached as shown in FIG. 1B or with the adapter 70 detached as shown in FIG. 1A. The adapter 70 is used for imaging narrow areas, such as between fingers or folds in an ear, as described later.

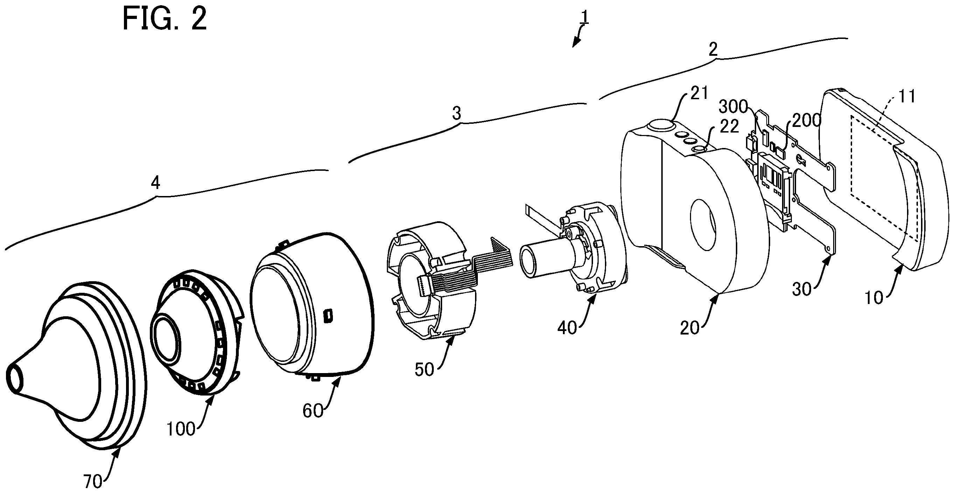

As shown in FIG. 2, the dermatoscopy camera 1 includes a controller 2, a camera body 3 in front of the controller 2, and an illumination device 4 in front of the camera body 3.

The controller 2 includes a display 10 that includes a touchscreen liquid crystal display (LCD) monitor 11 to display various items of operating information and captured images and receive, as setting means, user operations, a body 20 including operation buttons, such as a shutter button 21 and a power button 22, and a circuit board 30 housed between the display 10 and the body 20. The circuit board 30 includes a storage 200 that stores captured images and a control unit 300 that controls the components of the dermatoscopy camera 1.

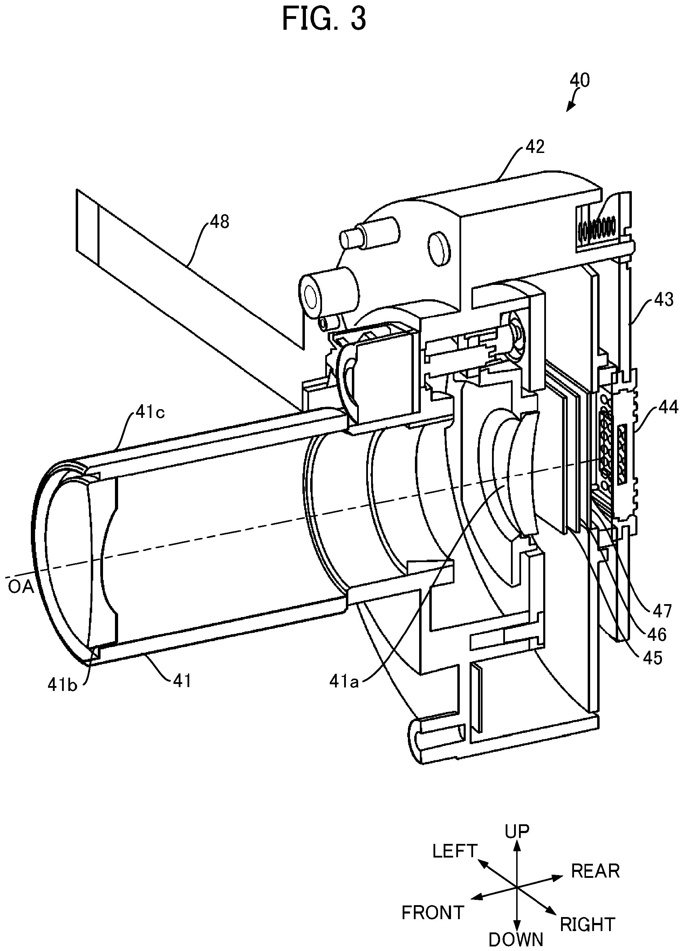

The camera body 3 includes an imager 40 and a frame 50. The frame 50 supports the imager 40 and is attached to a cover 60 (described later). FIG. 3 is a perspective cross-sectional view of the imager 40 included in the dermatoscopy camera 1. The imager 40 includes an imaging lens system 41 having a lens group aligned with an optical axis OA. The imager 40 also includes a wiring circuit board 43 and an image sensor 44 housed behind the imaging lens system 41. The imager 40 includes, in front of the image sensor 44, an infrared cut filter (IRCF) 45, an ultraviolet transmissive filter 46, and a polarization filter 47 in the stated order from the front. The imager 40 further includes a flexible circuit board 48 extending from the left of the imager 40. The controller 2 (FIG. 2) operates the imager 40 with the flexible circuit board 48.

The imaging lens system 41 includes the lens group including a first imaging lens 41a and a second imaging lens 41b aligned with the optical axis OA, and a lens barrel 41c housing the second imaging lens 41b. The first imaging lens 41a and the second imaging lens 41b are located between a diseased skin portion being an imaging target and the image sensor 44 and form an image of the imaging target on the image sensor 44. The first imaging lens 41a is movable in the front-rear direction, varying the focal length and thus varying magnification. The first imaging lens 41a and the second imaging lens 41b together magnify a diseased portion of a patient by 10 to 30 times.

The image sensor 44 is a known image sensor, such as a charge-coupled device (CCD) or a complementary metal-oxide semiconductor (CMOS). The image sensor 44 converts an optical image of a subject into an electrical signal. The imager 40 uses the image sensor 44 to image an imaging target. The imager 40 can capture, for example, still images and video.

As shown in FIG. 2, the illumination device 4 includes the cover 60 attached to the body 20 of the controller 2, an illumination device body 100 attached to the front end of the cover 60, and the adapter 70 attached to the cover 60 to cover the front of the illumination device body 100. As described above, the adapter 70 is detachable. The adapter 70 is attached to the cover 60 to image narrow areas, such as between fingers and folds in an ear, and detached to image other parts such as a face and an arm. Thus, the adapter 70 is a part of the illumination device 4 when used in imaging and is not a part of the illumination device 4 when unused in imaging.

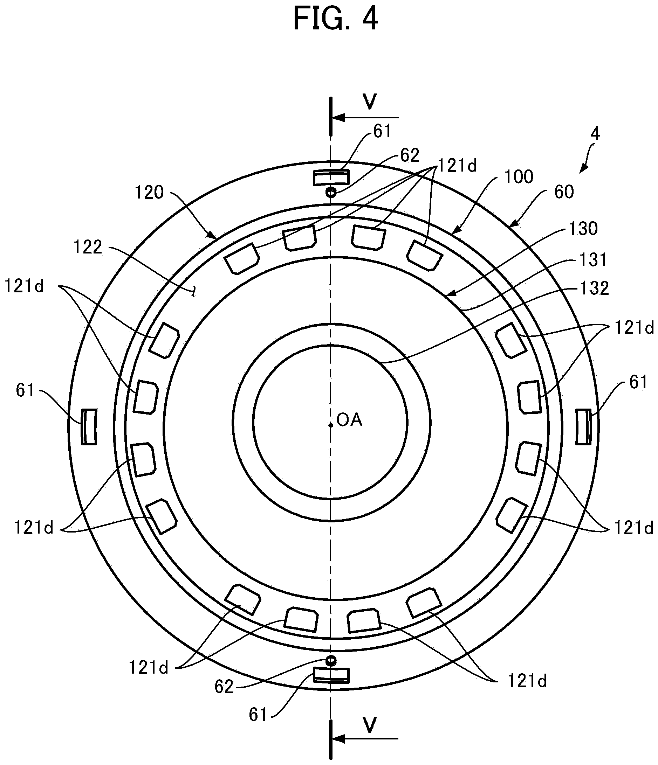

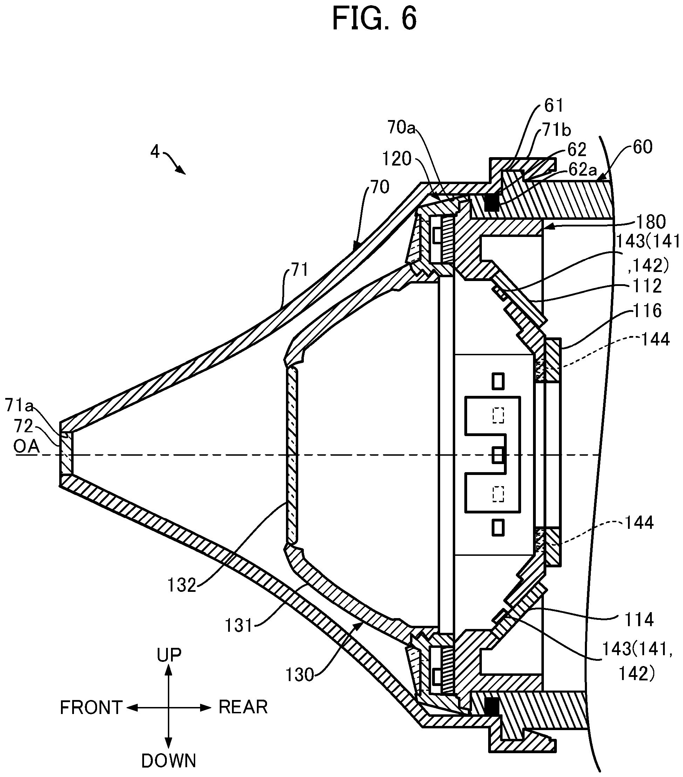

As shown in FIGS. 1A, 1B, 2, 4, and 5, the cover 60 is cylindrical and houses the camera body 3. As shown in FIG. 4, the cover 60 has four protrusions 61 arranged on the outer circumferential surface at equal intervals. The protrusions 61 engage with the adapter 70 when the adapter 70 is attached and disengage from the adapter 70 when the adapter 70 is detached. As shown in FIG. 4, the cover 60 also has two detection switches 62 arranged on the outer circumferential surface at equal intervals adjacent to two of the protrusions 61. The two detection switches 62 are arranged vertically opposite to each other with the optical axis OA between the detection switches 62 when the illumination device 4 is viewed from the front. As shown in FIG. 6, the detection switches 62 are urged by springs 62a in a direction to protrude from the cover 60. When the adapter 70 is detached, the detection switches 62 protrude from the cover 60 and are in an off state. When the adapter 70 is attached with the protrusions 61, the detection switches 62 are pressed by the adapter 70 and retracted. This turns on the detection switches 62. The two detection switches 62 function as detection means for detecting attachment and detachment of the adapter 70.

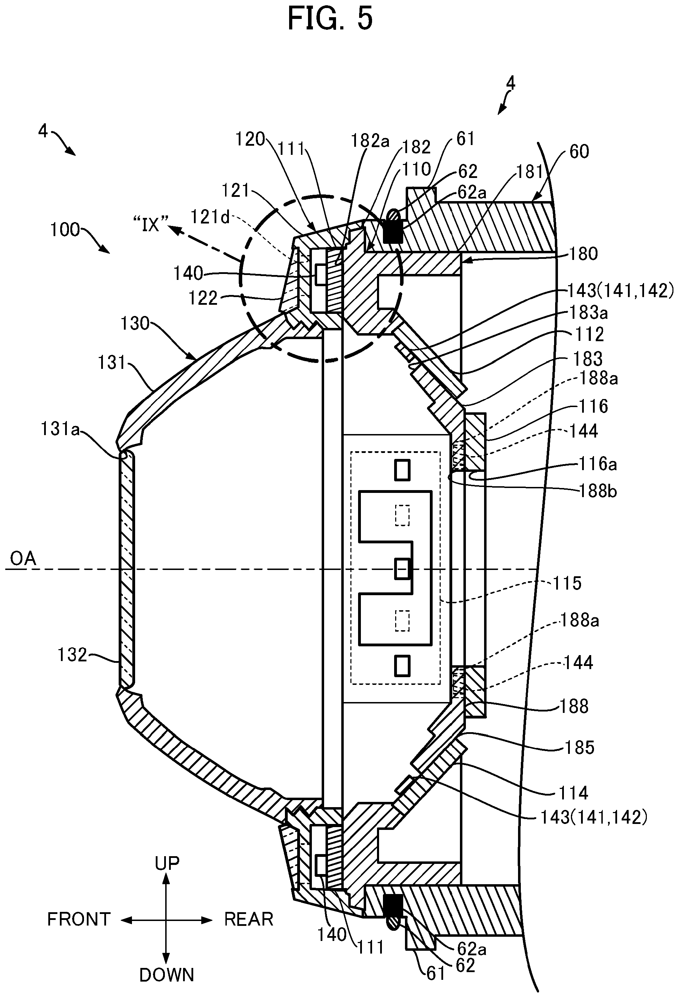

As shown in FIG. 5, the illumination device body 100 includes a base 110 provided with multiple light-emitting diode (LED) boards on which LEDs are mounted as light sources, an annular first cover 120 covering the periphery of the base 110, and a second cover 130 screwed to the first cover 120.

As shown in FIGS. 5, 7, and 8, the base 110 includes a base body 180, first to sixth LED boards 111 to 116 on the base body 180, and four polarizer plates 117 on the base body 180.

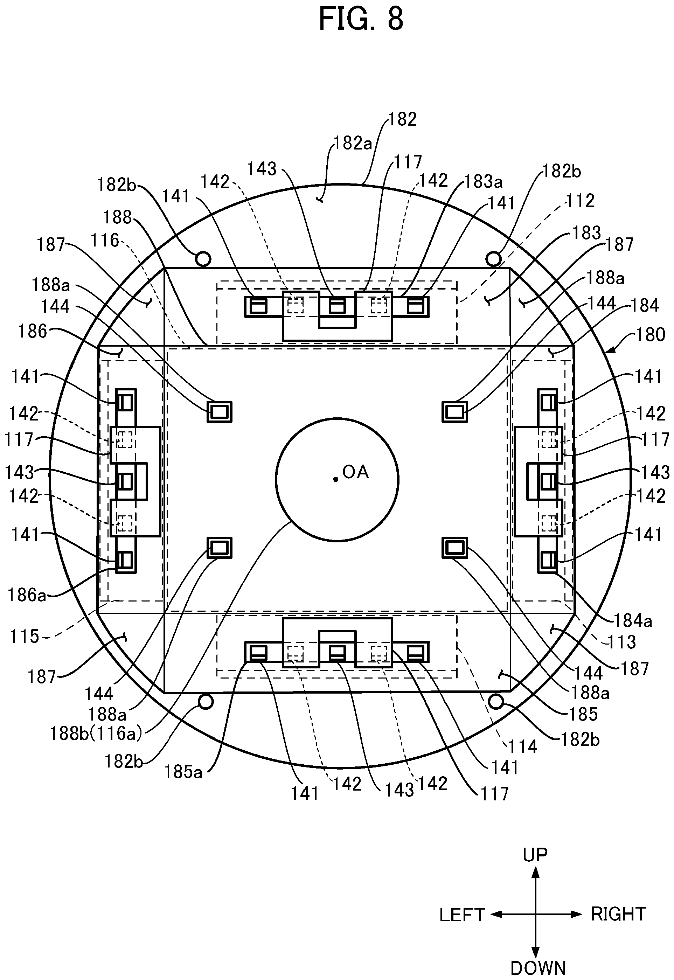

The base body 180 is formed from, for example, a black synthetic resin. As shown in FIGS. 5 and 8, the base body 180 includes a cylindrical portion 181, an annular portion 182 at the front end of the cylindrical portion 181, first to fourth walls 183 to 186 located radially inside the annular portion 182 and extending obliquely rearward from the annular portion 182, four connection parts 187 each between adjacent ones of the first to fourth walls 183 to 186, and a fifth wall 188 connected to edges of the first to fourth walls 183 to 186.

As shown in FIG. 5, the cylindrical portion 181 has an outer diameter that is substantially the same as the inner diameter of the cover 60 and has the outer circumferential surface fitted in the inner circumferential surface of the cover 60. This fastens the base body 180 to the cover 60.

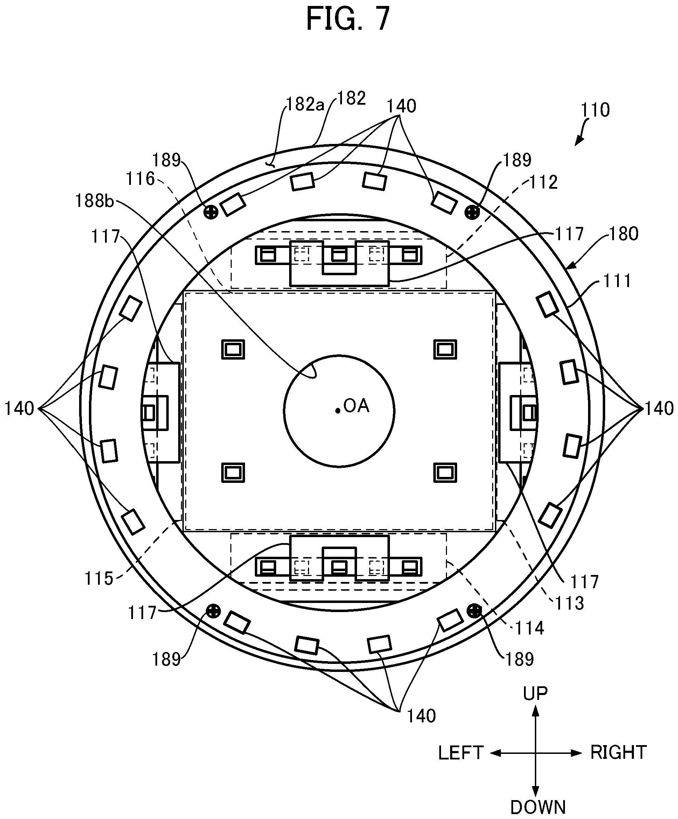

As shown in FIGS. 7 and 8, the annular portion 182 has a board mounting surface 182a on which the first LED board 111 is mounted. The board mounting surface 182a is flat and faces frontward. The board mounting surface 182a has four screw holes 182b used to mount the first LED board 111.

As shown in FIGS. 5 and 8, the first wall 183 has a rectangular-plate-like shape, with the main surface facing frontward and slightly downward and angled with the optical axis OA. The second LED board 112 is mounted on the first wall 183 from the rear thereof with screws (not shown), and the first wall 183 has an opening 183a to expose LEDs 141, 142, and 143 on the second LED board 112 frontward. A polarizer plate 117 is mounted on the front surface of the first wall 183 as shown in FIG. 8.

As shown in FIG. 8, the second wall 184 has a rectangular-plate-like shape, with the main surface facing frontward and slightly leftward and angled with the optical axis OA. The third LED board 113 is mounted on the second wall 184 from the rear side thereof with screws (not shown), and the second wall 184 has a opening 184a to expose LEDs 141, 142, and 143 on the third LED board 113 frontward. A polarizer plate 117 is mounted on the front surface of the second wall 184 as shown in FIG. 8.

As shown in FIGS. 5 and 8, the third wall 185 has a rectangular-plate-like shape, with the main surface facing frontward and slightly upward and angled with the optical axis OA. The fourth LED board 114 is mounted on the third wall 185 from the rear thereof with screws (not shown), and the third wall 185 has an opening 185a to expose LEDs 141, 142, and 143 on the fourth LED board 114 frontward. A polarizer plate 117 is mounted on the front surface of the third wall 185 as shown in FIG. 8.

As shown in FIG. 8, the fourth wall 186 has a rectangular shape, and has the main surface facing frontward and slightly rightward and angled with the optical axis OA. The fifth LED board 115 is mounted on the fourth wall 186 from the rear thereof with screws (not shown), and the fourth wall 186 has an opening 186a to expose LEDs 141, 142, and 143 on the fifth LED board 115 frontward. A polarizer plate 117 is mounted on the front surface of the fourth wall 186 as shown in FIG. 8.

The four connection parts 187 are each located between adjacent ones of the rectangular first to fourth walls 183 to 186 extending obliquely rearward from the annular portion 182. The four connection parts 187 thus connect the adjacent first to fourth walls 183 to 186 and form a circumferentially continuous wall together with the first to fourth walls 183 to 186.

As shown in FIGS. 5 and 8, the fifth wall 188 is a front-facing rectangular wall, and is connected to the edges of the first to fourth walls 183 to 186 extending rearward. The sixth LED board 116 is mounted on the fifth wall 188 from the rear thereof with screws (not shown), and the fifth wall 188 has openings 188a to expose four LEDs 144 on the sixth LED board 116 frontward. The fifth wall 188 has, at the center, a barrel insertion hole 188b for receiving the lens barrel 41c in the imager shown in FIG. 3.

The base body 180 holding the first to sixth LED boards 111 to 116 and the polarizer plates 117 is fitted to the cover 60 shown in FIG. 6.

As shown in FIGS. 5, 7, and 8, the first LED board 111 is annular and mounted to the board mounting surface 182a of the base body 180 with screws 189 (FIG. 7). The first LED board 111 includes sixteen LEDs 140 arranged circumferentially. The LEDs 140 emit, for example, white light and function as a ring flash that emits light frontward from the outer circumference of the illumination device body 100. The LEDs 140 emit light to illuminate a diseased skin portion when the dermatoscopy camera 1 (FIG. 1A) images the diseased skin portion as a common camera (normal imaging).

As shown in FIGS. 5 and 8, the second LED board 112 is a rectangular board screwed, from the rear, to the first wall 183 in the base body 180, and a longitudinal direction of the second LED board 112 extends laterally. As shown in FIG. 8, the second LED board 112 has an LED 141 that emits visible light, an LED 142 that emits visible light, an LED 143 that emits ultraviolet light, an LED 142 that emits visible light, and an LED 141 that emits visible light arranged in a row in this order from left to right. The two LEDs 142 are covered with the polarizer plate 117 and thus emit polarized light. As shown in FIG. 5, the second LED board 112 is located on the first wall 183 angled frontward with the vertical direction. The LEDs 141, 142, and 143 on the second LED board 112 thus face toward the optical axis OA of the imager 40, or more specifically, face frontward and slightly downward in FIG. 5.

As shown in FIG. 8, the third LED board 113 is a vertically-elongated rectangular board screwed, from the rear, to the second wall 184 in the base body 180 and extends orthogonally to the second LED board 112. As in the second LED board 112, the third LED board 113has an LED 141 that emits visible light, an LED 142 that emits visible light, an LED 143 that emits ultraviolet light, an LED 142 that emits visible light, and an LED 141 that emits visible light arranged in a row in this order from top to bottom. Also as in the second LED board 112, the two LEDs 142 are covered with the polarizer plate 117. The third LED board 113 is located on the angled second wall 184 in the base body 180. The LEDs 141, 142 and 143 on the third LED board 113 face toward the optical axis OA of the imager 40, or more specifically, face frontward and slightly leftward in FIG. 8.

As shown in FIG. 8, the fourth LED board 114 has the same structure as the second LED board 112 rotated by 180° about the optical axis OA. In other words, the LEDs 141, 142, and 143 on the second LED board 112 and the LEDs 141, 142, and 143 on the fourth LED board 114 are vertically symmetric with each other about the optical axis OA. The LEDs 141, 142, and 143 on the fourth LED board 114 face frontward and slightly upward and thus emit light toward the optical axis OA. The two LEDs 142 on the fourth LED board 114 are covered with the polarizer plate 117. As shown in FIG. 8, the fifth LED board 115 has the same structure as the third LED board 113 rotated by 180° about the optical axis OA. In other words, the LEDs 141, 142, and 143 on the third LED board 113 and the LEDs 141, 142, and 143 on the fifth LED board 115 are laterally symmetric with each other about the optical axis OA. The LEDs 141, 142, and 143 on the fifth LED board 115 face frontward and slightly rightward and thus emit light toward the optical axis OA. The two LEDs 142 on the fifth LED board 115 are covered with the polarizer plate 117. The LEDs 141, 142, and 143 on each of the second to fifth LED boards 112 to 115 illuminate a diseased skin portion during dermatoscopy imaging (first dermatoscopy imaging) performed with the adapter 70 (FIG. 6) detached from the illumination device 4 as shown in FIG. 5. The LEDs 142 and 143, except the LEDs 141, also illuminate a diseased skin portion during dermatoscopy imaging (second dermatoscopy imaging) performed with the adapter 70 attached to the illumination device 4 as shown in FIG. 6.

As shown in FIGS. 5 and 8, the sixth LED board 116 is a rectangular board screwed, from the rear, to the fifth wall 188 in the base body 180. The sixth LED board 116 has a barrel insertion hole 116a that aligns with the barrel insertion hole 188b in the fifth wall 188 in the base body 180. The sixth LED board 116 has the four LEDs 144 around the barrel insertion hole 188b (116a) to emit visible light during the second dermatoscopy imaging. The four LEDs 144 face frontward and emit light frontward through the openings 188a in the fifth wall 188 in the base body 180. The four LEDs 144 on the sixth LED board 116 illuminate a diseased skin portion instead of the LEDs 141 during the second dermatoscopy imaging.

The illumination device 4 thus includes the LEDs 140 that emit light during normal imaging, the LEDs 141 that emit light during the first dermatoscopy imaging alone, the LEDs 144 that emit light during the second dermatoscopy imaging alone, and the LEDs 142 and 143 that emit light during the first dermatoscopy imaging and the second dermatoscopy imaging.

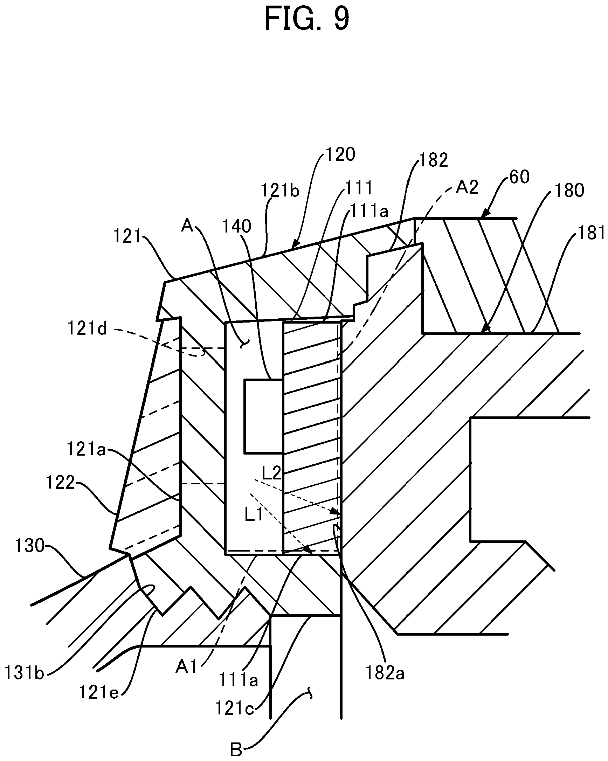

As shown in FIG. 5, the first cover 120 covering the periphery of the front end of the base 110 has a first cover body 121 and a translucent plate 122 on the front surface of the first cover body 121. The first cover body 121 is formed from, for example, a black synthetic resin. As shown in FIG. 9, the first cover body 121 has an annular cover portion 121a covering the front of the first LED board 111, and an outer wall 121b and an inner wall 121c forming a tubular shape and standing from the cover portion 121a. The first cover 120 is fastened to the base body 180 with the outer wall 121b and the inner wall 121c in contact with the annular portion 182. The first cover body 121 forms, on the rear, a housing space A housing the first LED board 111 and the LEDs 140. The housing space A is defined by the cover portion 121a, the outer wall 121b, the inner wall 121c, and the annular portion 182 in the base body 180. The cover portion 121a has light releasing holes aligned with the LEDs 140. The first cover 120 thus has sixteen light releasing holes 121d corresponding to the sixteen LEDs 140 on the first LED board 111 as shown in FIG. 4. The translucent plate 122 is formed from, for example, a transparent synthetic resin. The translucent plate 122 transmits light emitted from the LEDs 140 through the light releasing holes 121d and covers the light releasing holes 121d to prevent dust from entering the interior of the dermatoscopy camera 1. The illumination device 4 can thus function as a ring flash by emitting light from the LEDs 140 through the multiple light releasing holes 121d arranged circumferentially.

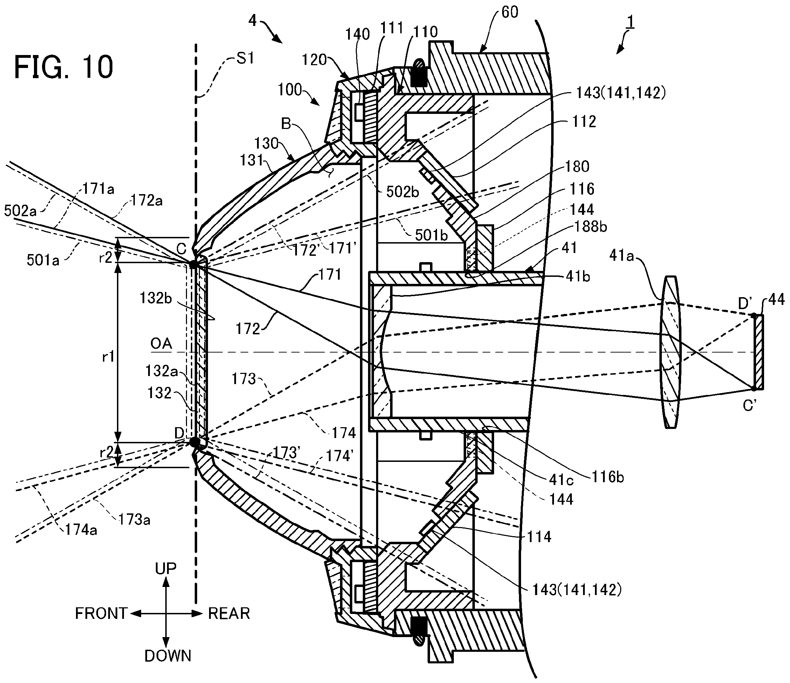

As shown in FIG. 9, the outer wall 121b of the first cover body 121 covers the radially outer end face 111a of the first LED board 111, and the inner wall 121c covers the radially inner end face 111a. As described above, the base body 180 and the first cover body 121 defining the housing space A are formed from, for example, a black synthetic resin. The base body 180, the outer wall 121b, and the inner wall 121c thus absorb light from the LEDs 140 transmitted through the first LED board 111. For example, the inner wall 121c absorbs light L1 transmitted through the first LED board 111 before the light L1 passes through the inner end face 111a. Also, the base body 180 absorbs light L2 transmitted through the first LED board 111 before the light L2 travels further rearward. Thus, the light emitted from the LEDs 140 travels outward from the housing space A through the light releasing holes 121d alone, thus reducing light leakage to the interior of the dermatoscopy camera 1 (for example, to a space B housing the distal end of the lens barrel 41c and the LEDs 141, 142, 143, and 144 for dermatoscopy imaging as shown in FIG. 10). As shown in FIG. 9, the inner wall 121c has internal threads 121e for engaging with the second cover 130.

As shown at least in FIGS. 4 to 6, the second cover 130 includes a truncated conical cylinder 131 as a first cover, and a first objective cover 132 fitted in an opening 131a at the top (front end) of the cylinder 131. The cylinder 131 is formed from a light non-transmissive resin material, such as a polyvinyl chloride derivative and an acrylic resin, to avoid light transmission and conversion of light into an electrical signal by the image sensor. The cylinder 131 may have a surface coated with a paint that prevents light transmission. The cylinder 131 has an inner circumferential surface embossed to reduce light reflection or treated with an anti-reflective coating. As shown in FIG. 9, the cylinder 131 has, at the bottom (rear end), external threads 131b screwed with the internal threads 121e on the inner wall 121c. The external threads 131b and the internal threads 121e on the first cover 120 function as attachment and detachment means for attaching and detaching the second cover 130 to and from the first cover 120.

The first objective cover 132 shown in FIG. 5 is a member that can transmit light, such as a glass piece, and is disk-shaped. The first objective cover 132 has the main surface orthogonal to the optical axis OA. The main surface of the first objective cover 132 has a predetermined first area. Dermatoscopy imaging of a diseased skin portion is to be performed with less diffuse reflection of light on the skin surface. Diffuse reflection of light is reduced with a gel applied to the diseased skin portion against which the first objective cover 132 is pressed. This removes an air layer between the diseased skin portion and the first objective cover 132, thus reducing diffuse reflection of light on the skin surface. In the manner described above, the first objective cover 132 pressed against the diseased skin portion transmits light from the LEDs 141, 142, 143, and 144 mounted on the second to sixth LED boards 112 to 116 shown in FIG. 8 allows illumination of the diseased skin portion with light, and directs light reflected by the diseased skin portion into the dermatoscopy camera 1 and thus to the imager 40.

During the first dermatoscopy imaging, as shown in FIG. 10, the first objective cover 132 is in contact with skin S1 in a region r1. The region r1 is surrounded by a region r2 in which the top of the cylinder 131 is in contact with the skin S1. The cylinder 131 in contact with the skin S1 in the region r2 blocks external light. The skin S1 in contact with the first objective cover 132 is thus less susceptible to external light. This allows the skin S1 in contact with the first objective cover 132 to be efficiently illuminated with light from the LEDs 141, 142, 143, and 144 on the second to sixth LED boards 112 to 116. The first objective cover 132 pressed against the skin S1 retains a constant distance between the skin S1 and the dermatoscopy camera 1 and constant brightness at the illuminated skin S1, thus enabling stable dermatoscopy imaging under, for example, stable exposure conditions. Additionally, the first objective cover 132 protects the interior of the dermatoscopy camera 1 from moisture and dust.

As shown in FIGS. 1B and 6, the adapter 70 includes a truncated conical cylinder 71 as a second cover and a second objective cover 72 fitted in an opening 71a at the top (front end) of the cylinder 71. The cylinder 71 is formed from, for example, a resin such as a polyvinyl chloride derivative and an acrylic resin, and has an inner circumferential surface embossed to reduce light reflection or treated with an anti-reflective coating. The cylinder 71 has a hooked portion 71b at the bottom (rear end) as shown in FIG. 6. The hooked portion 71b is engaged with the protrusions 61 on the cover 60 to attach the adapter 70 to the cover 60. This causes an inner wall 70a of the adapter 70 to press the detection switches 62. The detection switches 62 are then retracted and turned on. When the adapter 70 is detached from the cover 60, the hooked portion 71b is pushed outward to be disengaged from the protrusions 61. This releases the detection switches 62 from the state pressed by the inner wall 70a of the adapter 70. The detection switches 62 then protrude from the cover 60 and are turned off. The hooked portion 71b and the protrusions 61 function as attachment and detachment means for attaching and detaching the adapter 70 to and from the cover 60.

The second objective cover 72 is a member that can transmit light, such as a glass piece, and is disk-shaped. During the second dermatoscopy imaging, the second objective cover 72 comes in contact with a diseased skin portion as an imaging target. The second objective cover 72 has the main surface orthogonal to the optical axis OA. The main surface of the second objective cover 72 has a predetermined second area that is smaller than the first area of the first objective cover 132. The second objective cover 72 can thus be placed on a diseased skin portion in a narrow area with the tapered cylinder 71. The second objective cover 72 is pressed against a diseased skin portion in a narrow area through a gel applied to the diseased skin portion to reduce diffuse reflection of light on the diseased skin portion. During the second dermatoscopy imaging, the second objective cover 72 transmits light emitted from the LEDs 142 and 143 on the second to fifth LED boards 112 to 115 and from the LEDs 144 on the sixth LED board 116 shown in FIG. 8 to illuminate the diseased skin portion with light, and allows light reflected by the diseased skin portion to enter the second objective cover 72.

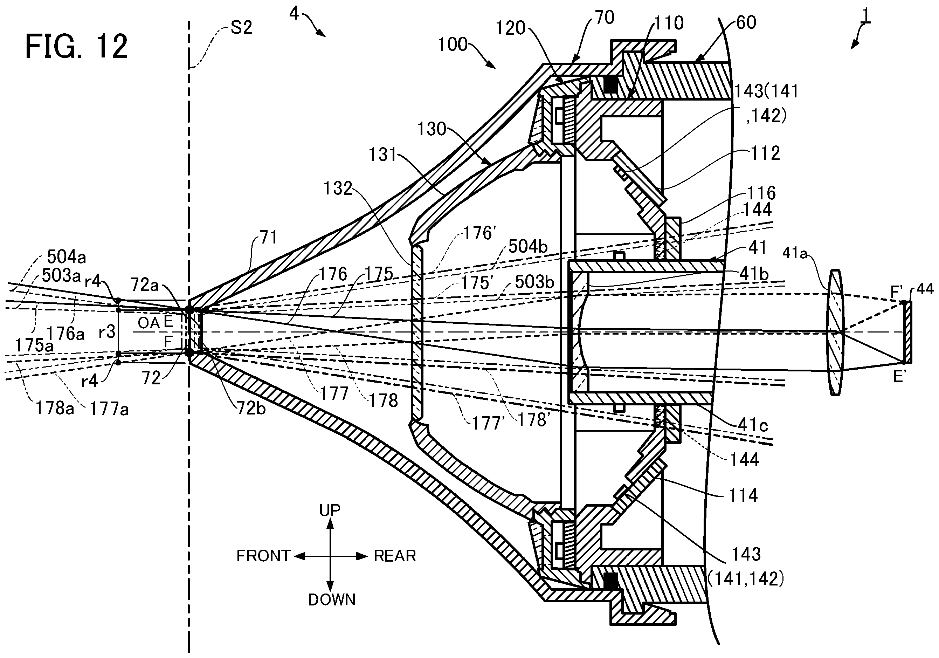

During the second dermatoscopy imaging, as shown in FIG. 12, the second objective cover 72 is in contact with skin S2 in a region r3. The region r3 is surrounded by a region r4 in which the top of the cylinder 71 is in contact with the skin S2. The cylinder 71 in contact with the skin S2 in the region r4 blocks external light. The skin S2 in contact with the second objective cover 72 is thus less susceptible to external light. The second objective cover 72 enables stable dermatoscopy imaging as the same advantageous effects as produced by the first objective cover 132 described above.

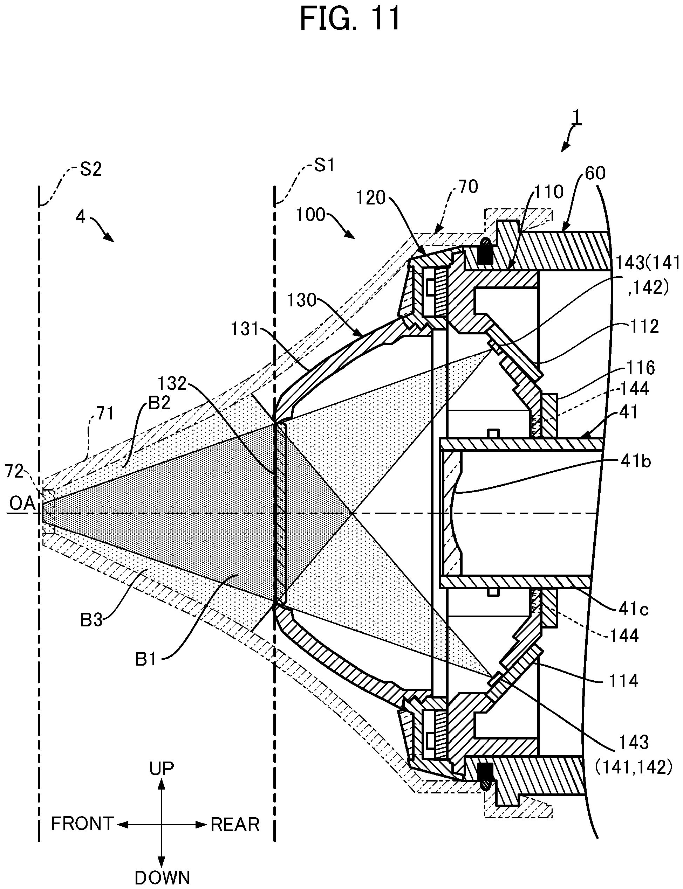

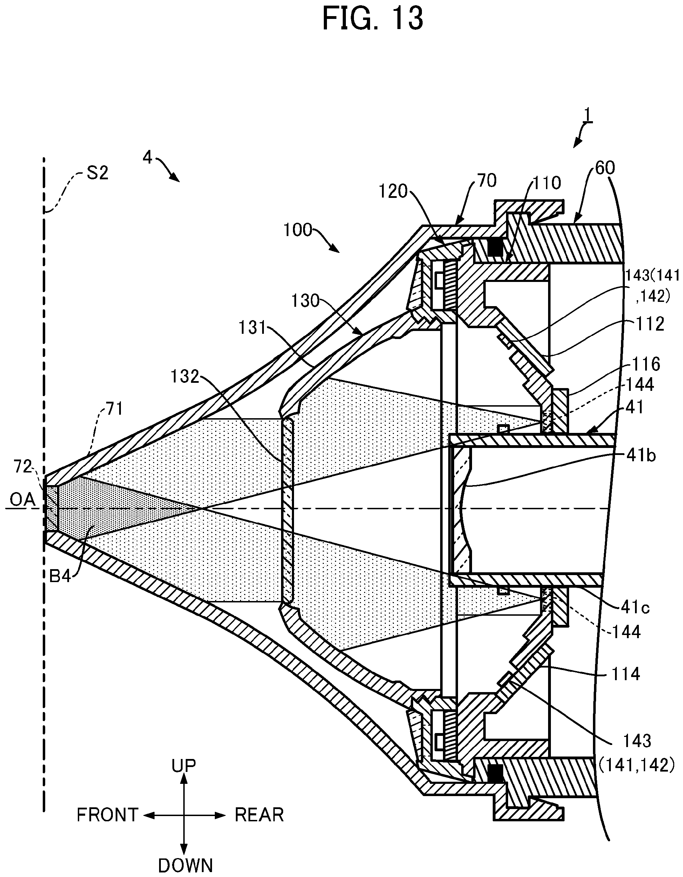

Referring now to FIGS. 10 to 13, the LEDs, each of which is an example of light emitting means in the illumination device 4, are arranged in the manner described below in more detail. In FIGS. 10 to 13, the front portion of the dermatoscopy camera 1 is illustrated to simplify the drawings. FIGS. 10 to 13 are cross-sectional views of the dermatoscopy camera 1 taken along a vertical line aligned with the line along which the cross-sectional views in FIGS. 5 and 6 are taken. In FIGS. 11 and 13, the range illuminated by the LEDs is hatched, with an area illuminated brighter being hatched darker.

As shown in FIG. 10, the imaging lens system 41 in the dermatoscopy camera 1 includes the lens barrel 41c placed through the barrel insertion holes 116a and 188b and protruding from the base body 180. During the first dermatoscopy imaging, the imaging lens system 41 moves the lens group to focus on the skin S1 in contact with the first objective cover 132. FIG. 10 shows the path of light that enters the dermatoscopy camera 1 and then the image sensor 44 through the lens group, such as the first imaging lens 41a, assuming that the skin S1 is in contact with the first objective cover 132.

The skin S1 includes a point C at the uppermost position of the imaging range of the image sensor 44. Thus, rays passing through the point C during the first dermatoscopy imaging are at the uppermost (outermost) position among the rays included in the pencil of rays that enters the image sensor 44 through the first objective cover 132 from the imaging target. Light reflected at the point C on the skin S1 travels in various directions and partially enters the image sensor 44 through the lens group. Among the rays passing through the point C and entering the image sensor 44, an upper ray 171 is the uppermost ray and a lower ray 172 is the lowermost ray. The upper ray 171 is also the uppermost (outermost) ray among the multiple upper rays that pass through the first objective cover 132 and enter the image sensor 44. The upper ray 171 and the lower ray 172 passing through the point C are transmitted through the first objective cover 132, refracted by the lens group, including the second imaging lens 41b and the first imaging lens 41a, and focused at a point C' at an end of the image sensor 44. Among the rays passing through the point C, rays between the upper ray 171 and the lower ray 172 (e.g., principal rays) are also transmitted through the first objective cover 132, refracted by the lens group, such as the second imaging lens 41b, and focused at the point C' at the end of the image sensor 44.

The first objective cover 132 in contact with the skin S1 during the first dermatoscopy imaging allows little or no light to enter from the front. Thus, the upper ray 171 and the lower ray 172 have the portions frontward (nearer the skin S1) from the first objective cover 132 being imaginary and may thus have the portions referred to as an imaginary upper ray 171a and an imaginary lower ray 172a.

The upper ray 171 passing through the point C has a portion (imaginary upper ray 171a) frontward (nearer the skin S1) from the first objective cover 132. An inverted upper ray 171' is an imaginary ray that is obtained by inverting the portion 171a toward the image sensor 44 with respect to a symmetry axis being a line extending vertically (orthogonally to the optical axis OA) along the front surface 132a of the first objective cover 132. The lower ray 172 passing through the point C has a portion (imaginary lower ray 172a) frontward (nearer the skin S1) from the first objective cover 132. An inverted lower ray 172' is an imaginary ray that is obtained by inverting the portion 172a toward the image sensor 44 with respect to the symmetry axis being the line extending vertically along the front surface 132a of the first objective cover 132.

The skin S1 includes a point D at the lowermost position of the imaging range of the image sensor 44. Thus, rays passing through the point D during the first dermatoscopy imaging are at the lowermost (outermost) position among the rays included in the pencil of rays that enters the image sensor 44 through the first objective cover 132 from the imaging target. Light reflected at the point D on the skin S1 travels in various directions and partially enters the image sensor 44 through the lens group. Among the rays passing through the point D and entering the image sensor 44, an upper ray 173 is the uppermost ray and a lower ray 174 is the lowermost ray. The lower ray 174 is also the lowermost (outermost) ray among the multiple lower rays that enter the image sensor 44 through the lens group. The upper and lower rays 173 and 174 are indicated with dashed lines for easy distinction from the upper and lower rays 171 and 172 indicated with solid lines. The upper ray 173 and the lower ray 174 passing through the point D are transmitted through the first objective cover 132, refracted by the lens group (e.g., the second imaging lens 41b), and focused at a point D' at another end of the image sensor 44. Among the rays passing through the point D, rays between the upper ray 173 and the lower ray 174 (e.g., principal rays) are also transmitted through the first objective cover 132, refracted by the lens group, and focused at the point C' at the end of the image sensor 44.

The first objective cover 132 in contact with the skin S1 during the first dermatoscopy imaging allows little or no light to enter from the front. Thus, the upper ray 173 and the lower ray 174 have the portions frontward (nearer the skin S1) from the first objective cover 132 being imaginary and may thus have the portions referred to as an imaginary upper ray 173a and an imaginary lower ray 174a.

The upper ray 173 passing through the point D has a portion (imaginary upper ray 173a) frontward (nearer the skin S1) from the first objective cover 132. An inverted upper ray 173' is an imaginary ray that is obtained by inverting the portion 173a toward the image sensor 44 with respect to the symmetry axis being the line extending vertically (orthogonally to the optical axis OA) along the front surface 132a of the first objective cover 132. The lower ray 174 passing through the point D has a portion (imaginary lower ray 174a) frontward (nearer the skin S1) from the first objective cover 132. An inverted lower ray 174' is an imaginary ray that is obtained by inverting the portion 174a toward the image sensor 44 with respect to the symmetry axis being the line extending vertically along the front surface 132a of the first objective cover 132.

The upper ray 171 and the lower ray 174, being the uppermost ray and the lowermost ray in the pencil of rays that enters the image sensor 44 through the first objective cover 132, define a region between the rays 171 and 174, including a first region 501a located frontward from the first objective cover 132. The first region 501a inverted with respect to the symmetry axis being the line extending vertically (orthogonally to the optical axis OA) along the front surface 132a of the first objective cover 132 is a first inverted region 501b. The first region 501a is an imaginary region between the imaginary upper ray 171a and the imaginary lower ray 174a. The first inverted region 501b is also an imaginary region between the inverted upper rays 171' and the inverted lower rays 174'. Although the first region 501a and the first inverted region 501b each have a trapezoidal cross section, these region 501a and 501b are both actually truncated conical, with the first region 501a defined by the imaginary upper ray 171a and the imaginary lower ray 174a being rotated about the optical axis OA and the first inverted region 501b defined by the first region 501a being inverted. The LEDs 141, 142, and 143 on the second LED board 112 and the fourth LED board 114 are outward from the first inverted region 501b, as viewed from the optical axis OA. The LEDs 141, 142, and 143 arranged in the above manner emit light that is reflected by the rear surface 132b of the first objective cover 132 and restricted from entering the image sensor 44. This prevents the central area of a captured image from being affected by light from the LEDs 141, 142, and 143 reflected by the rear surface 132b of the first objective cover 132 and reduces the likelihood of imaging being affected by the reflected light.

Among the rays entering the image sensor 44 and included in the portion of the pencil of rays frontward from the first objective cover 132, the outermost rays are the imaginary lower ray 172a and the imaginary upper ray 173a. The imaginary lower ray 172a and the imaginary upper ray 173a define a region between the rays 172a and 173a, including a second region 502a located frontward from the first objective cover 132. The second region 502a inverted with respect to the symmetry axis being the line extending vertically (orthogonally to the optical axis OA) along the front surface 132a of the first objective cover 132 is a second inverted region 502b. The second region 502a is an imaginary region between the imaginary lower ray 172a and the imaginary upper ray 173a. The second inverted region 502b is also an imaginary region between the inverted lower ray 172' and the inverted upper ray 173'. Although the second region 502a and the second inverted region 502b each have a trapezoidal cross section, they are both actually truncated conical, with the second region 502a defined by the imaginary lower ray 172a and the imaginary upper ray 173a being rotated about the optical axis OA and the second inverted region 502b defined by the second region 502a being inverted. The LEDs 141, 142, and 143 on the second LED board 112 and the fourth LED board 114 are within the second inverted region 502b. The LEDs 141, 142 and 143 are thus unlikely to be located largely outward to avoid upsizing the illumination device 4 and the dermatoscopy camera 1.

The LEDs 141, 142 and 143 on the vertically arranged second LED board 112 and the fourth LED board 114 are arranged in the manner described above with reference to the cross-sectional view (FIG. 10) taken along a vertical line. The LEDs 141, 142 and 143 on the laterally arranged third LED board 113 (FIG. 8) and the fifth LED board 115 (FIG. 8) can also be arranged in the same manner and may be described with reference to a cross-sectional view taken along a lateral line. More specifically, the LEDs 141, 142, and 143 on the third LED board 113 (FIG. 8) and the fifth LED board 115 (FIG. 8) are also outward from the first inverted region 501b and within the second inverted region 502b as viewed from the optical axis OA.

As shown in FIG. 11, the LEDs 141, 142, and 143 on the second LED board 112 face frontward and slightly downward. Similarly, the LEDs 141, 142, and 143 on the fourth LED board 114 face frontward and slightly upward as shown in FIG. 11. As shown in FIG. 8, the LEDs 141, 142, and 143 on the third LED board 113 face frontward and slightly leftward, and the LEDs 141, 142, 143 on the fifth LED board 115 face frontward and slightly rightward. The LED 141, 142, and 143 arranged on the second to fifth LED boards 112 to 115 in the above manner thus emit light toward the first objective cover 132 during the first dermatoscopy imaging. The first objective cover 132 can thus be entirely located within a brightly illuminated space B1, allowing the skin S1 in contact with the first objective cover 132 to be illuminated brightly with reduced unevenness.

As described above, the light emitted from each of the LEDs 141, 142, and 143 is directed toward the first objective cover 132 during the first dermatoscopy imaging. This allows the skin S1 to be illuminated brightly with reduced unevenness. The LEDs 141, 142, and 143 are arranged in the manner described above to prevent the central area of the captured image from being affected by light from the LEDs 141, 142, and 143 reflected by the first objective cover 132 and to reduce the likelihood of imaging being affected by the reflected light, while avoiding upsizing the illumination device 4 and the dermatoscopy camera 1.

The LEDs 144 that emit light during the second dermatoscopy imaging and are arranged on the sixth LED board 116 will now be described. As shown in FIG. 12, during the second dermatoscopy imaging, the imaging lens system 41 moves the lens group to focus on the skin S2 in contact with the second objective cover 72. FIG. 12 shows the path of light that enters the dermatoscopy camera 1 and then the image sensor 44 through the lens group, such as the first imaging lens 41a, when the skin S2 is in contact with the second objective cover 72.

The skin S2 includes a point E at the uppermost position of the imaging range of the image sensor 44. Thus, rays passing through the point E during the second dermatoscopy imaging are at the uppermost (outermost) position among the rays included in the pencil of rays that enters the image sensor 44 through the second objective cover 72 from the imaging target. Light reflected at the point E on the skin S2 travels in various directions and partially enters the image sensor 44 through the lens group. Among the rays passing through the point E and entering the image sensor 44, an upper ray 175 is the uppermost ray and a lower ray 176 is the lowermost ray. The upper ray 175 is also the uppermost (outermost) ray among the multiple upper rays that pass through the second objective cover 72 and enter the image sensor 44. The upper ray 175 and the lower ray 176 passing through the point E are transmitted through the second objective cover 72 and the first objective cover 132, refracted by the lens group, and focused at a point E' at an end of the image sensor 44.

The second objective cover 72 in contact with the skin S2 during the second dermatoscopy imaging allows little or no light to enter from the front. Thus, the upper ray 175 and the lower rays 176 have the portions frontward (nearer the skin S2) from the second objective cover 72 being imaginary and may thus have the portions referred to as an imaginary upper ray 175a and an imaginary lower ray 176a.

The upper ray 175 passing through the point E has a portion (imaginary upper ray 175a) frontward (nearer the skin S2) from the second objective cover 72. An inverted upper ray 175' is an imaginary ray that is obtained by inverting the portion 175a toward the image sensor 44 with respect to a symmetry axis being a line extending vertically (orthogonally to the optical axis OA) along the front surface 72a of the second objective cover 72. The lower ray 176 passing through the point E has a portion (imaginary lower ray 176a) frontward (nearer the skin S2) from the second objective cover 72. An inverted lower ray 176' is an imaginary ray that is obtained by inverting the portion 176a toward the image sensor 44 with respect to the symmetry axis being the line extending vertically along the front surface 72a of the second objective cover 72.

The skin S2 includes a point F at the lowermost position of the imaging range of the image sensor 44. Thus, rays passing through the point F during the second dermatoscopy imaging are at the lowermost (outermost) position among the rays included in the pencil of rays that enters the image sensor 44 through the second objective cover 72 from the imaging target. Light reflected at the point F on the skin S2 travels in various directions and partially enters the image sensor 44 through the lens group. Among the rays passing through the point E and entering the image sensor 44, an upper ray 177 is the uppermost ray and a lower ray 178 is the lowermost ray. The lower ray 178 is also the lowermost (outermost) ray among the multiple lower rays that pass through the second objective cover 72 and enter the image sensor 44. The upper ray 177 and the lower ray 178 passing through the point F are transmitted through the second objective cover 72 and the first objective cover 132, refracted by the lens group, and focused at a point F' at another end of the image sensor 44.

The second objective cover 72 in contact with the skin S2 during the second dermatoscopy imaging allows little or no light to enter from the front. Thus, the upper ray 177 and the lower ray 178 have the portions frontward (nearer the skin S2) from the second objective cover 72 being imaginary and may thus have the portions referred to as an imaginary upper ray 177a and an imaginary lower ray 178a.

The upper ray 177 passing through the point F has a portion (imaginary upper ray 177a) frontward (nearer the skin S2) from the second objective cover 72. An inverted upper ray 177' is an imaginary ray that is obtained by inverting the portion 177a toward the image sensor 44 with respect to the symmetry axis being the line extending vertically (orthogonally to the optical axis OA) along the front surface 72a of the second objective cover 72. The lower ray 178 passing through the point F has a portion (imaginary lower ray 178a) frontward (nearer the skin S2) from the second objective cover 72. An inverted lower ray 178' is an imaginary ray that is obtained by inverting the portion 178a toward the image sensor 44 with respect to the symmetry axis being the line extending vertically along the front surface 72a of the second objective cover 72.

The upper ray 175 and the lower ray 178, being the uppermost ray and the lowermost ray in the pencil of rays that enters the image sensor 44 through the second objective cover 72, define a region between the rays 175 and 178, including a third region 503a located frontward from the second objective cover 72. The third region 503a inverted with respect to the symmetry axis being the line extending vertically (orthogonally to the optical axis OA) along the front surface 72a of the second objective cover 72 is a third inverted region 503b. The third region 503a is an imaginary region between the imaginary upper ray 175a and the imaginary lower ray 178a. The third inverted region 503b is also an imaginary region between the inverted upper ray 175' and the inverted lower ray 178'. Although the third inverted region 503a and the third inverted region 503b each have a trapezoidal cross section, they are both actually truncated conical, with the third inverted region 503a defined by the imaginary upper ray 175a and the imaginary lower ray 178a being rotated about the optical axis OA and the third inverted region 503b defined by the third region 503a being inverted. The LEDs 144 on the sixth LED board 116 are outward from the third inverted region 503b. The LEDs 144 on the sixth LED board 116 are arranged in the same manner as in a cross-sectional view taken along a lateral line. The LEDs 144 are outward from the third inverted region 503b as viewed from the optical axis OA. The LEDs 144 arranged in the above manner emit light that is reflected by the rear surface 72b of the second objective cover 72 and restricted from entering the image sensor 44. This prevents the central area of a captured image from being affected by light from the LEDs 144 reflected by the rear surface 72b of the second objective cover 72 and reduces the likelihood of imaging being affected by the reflected light.

Among the rays entering the image sensor 44 and included in the portion of the pencil of rays frontward from the first objective cover 72, the outermost rays are the imaginary lower ray 176a and the imaginary upper ray 177a. The imaginary lower ray 176a and the imaginary upper ray 177a define a region between the rays 176a and 177a, including a fourth region 504a located frontward from the second objective cover 72. The fourth region 504a inverted with respect to the symmetry axis being the line extending vertically (orthogonally to the optical axis OA) along the front surface 72a of the second objective cover 72 is a fourth inverted region 504b. The fourth region 504a is an imaginary region between the imaginary lower ray 176a and the imaginary upper ray 177a. The fourth inverted region 504b is also a region between the inverted lower ray 176' and the inverted upper ray 177'. Although the fourth region 504a and the fourth inverted region 504b each have a trapezoidal cross section, they are both actually truncated conical, with the fourth region 504a defined by the imaginary lower ray 176a and the imaginary upper ray 177a being rotated about the optical axis OA and the fourth inverted region 504b defined by the fourth region 504a being inverted. The LEDs 144 on the sixth LED board 116 are within the fourth inverted region 504b. The LEDs 144 on the sixth LED board 116 are arranged in the same manner as in a cross-sectional view taken along a lateral line. The LEDs 144 are within the fourth inverted region 504b. This allows the LEDs 144 to illuminate the second objective cover 72 from nearer the optical axis OA, reducing unevenness of the light illuminated on the skin S2.

As described above, the four LEDs 144 face frontward. As shown in FIG. 13, the light emitted frontward from each of the four LEDs 144 during the second dermatoscopy imaging is directed toward the second objective cover 72. The second objective cover 72 is thus entirely located within a brightly illuminated space B4, allowing the skin S2 in contact with the second objective cover 72 to be illuminated brightly with reduced unevenness.

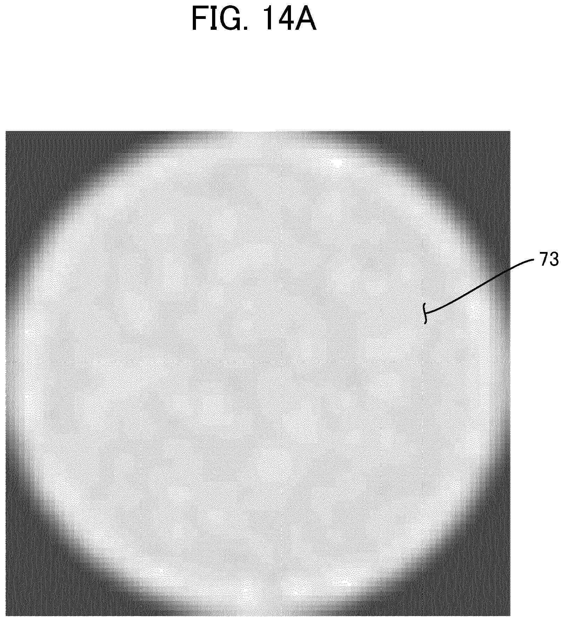

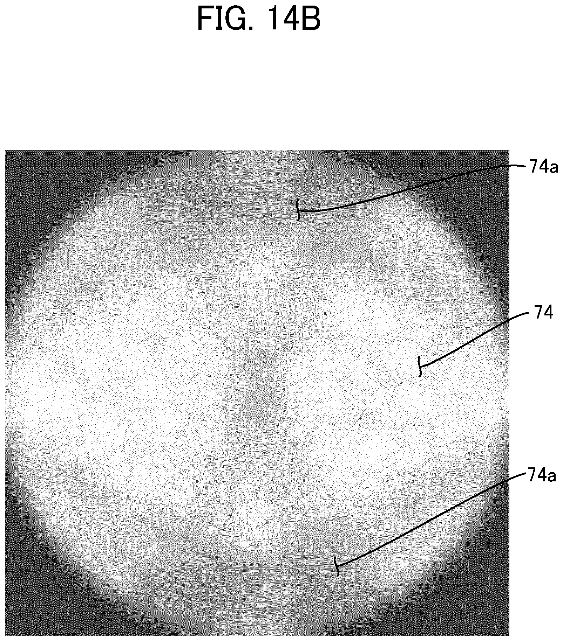

As described above, although the dermatoscopy camera 1 emits visible light with the LEDs 141 during the first dermatoscopy imaging, the dermatoscopy camera 1 located nearer the optical axis OA emits visible light with the LEDs 144 during the second dermatoscopy imaging. This facilitates illuminating the second objective cover 72 with a smaller diameter that is coaxial with the optical axis OA. This is also demonstrated by the simulation of the illuminance distribution for the second objective cover as shown in FIGS. 14A and 14B. In FIG. 14A, the LEDs 144 shown in FIG. 8 emit light. In FIG. 14B, the eight LEDs 141 shown in FIG. 8 emit light. In FIG. 14A, the second objective cover has an area 73 illuminated uniformly and brightly with reduced unevenness. In FIG. 14B, the second objective cover has an area 74 partially including a darker area 74a illuminated insufficiently, causing uneven brightness.

The second objective cover 72 may cause such uneven brightness when illuminated with the LEDs 141 (FIG. 8) during the first dermatoscopy imaging. This may result from the LEDs 141 for the first dermatoscopy imaging arranged to emit light toward the first objective cover 132 as described above. As shown in FIG. 11, light emitted from one of the LEDs 141 passing through the first objective cover 132 partially illuminates the second objective cover 72 instead of entirely illuminating the second objective cover 72. Thus, part of the second objective cover 72 is located in the brightly illuminated space B1 whereas the remaining part is located in spaces B2 and B3 darker than the space B1. This can cause uneven brightness on the second objective cover 72. Such uneven brightness is caused markedly by the LEDs 141 that are farthest from the optical axis OA among the LEDs 141, 142, and 143 that emit light during the first dermatoscopy imaging.

The LEDs 144 for the second dermatoscopy imaging are located nearer the optical axis OA than the LEDs 141 for the first dermatoscopy imaging. This arrangement enables the second objective cover 72 to be illuminated uniformly with light emitted frontward from the LEDs 144 as shown in FIG. 13. This reduces uneven brightness in the images resulting from the second dermatoscopy imaging and enables capturing of images that facilitate diagnosis.

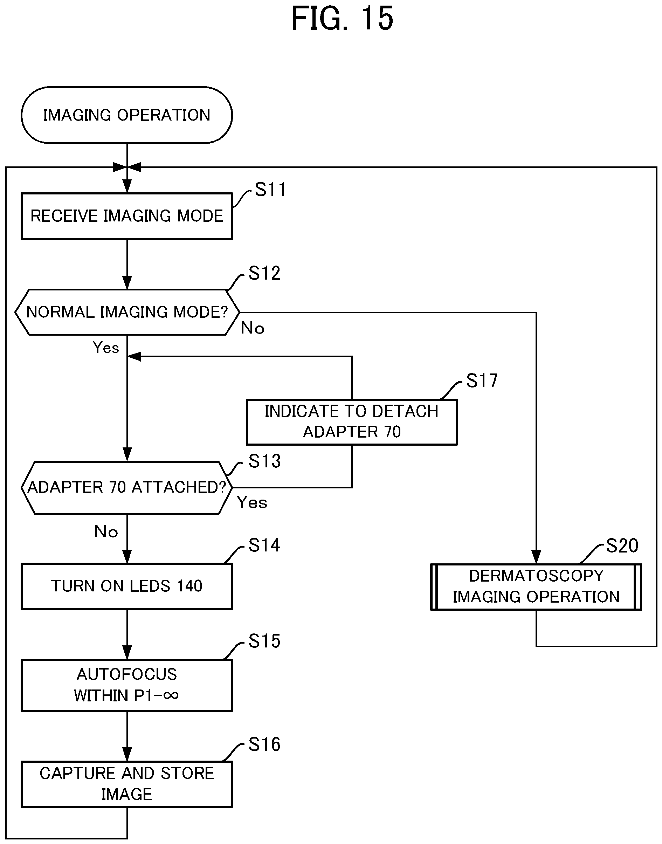

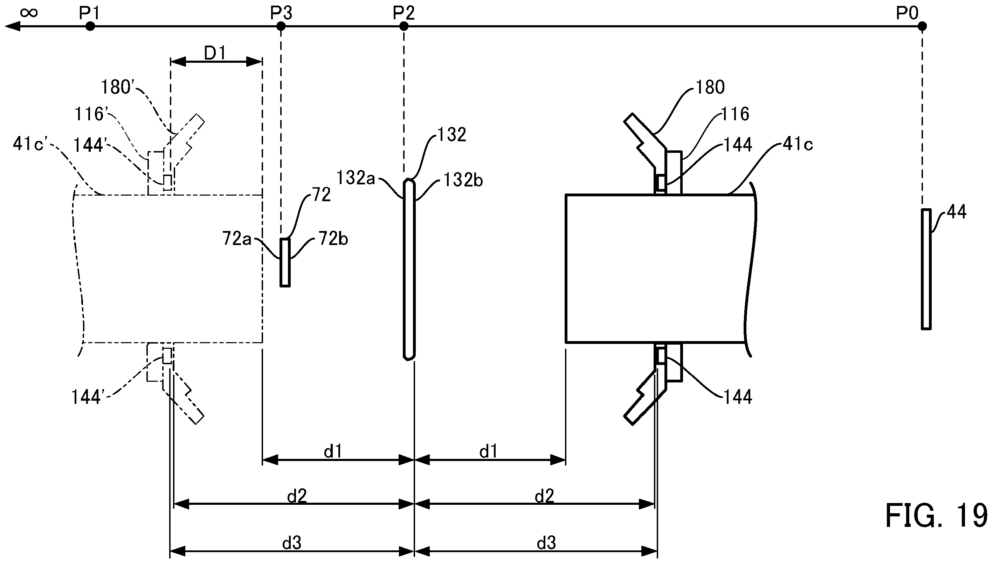

The operation of the dermatoscopy camera 1 will now be described with reference to FIGS. 15 to 19. For ease of understanding, FIG. 19 simply shows the components of the dermatoscopy camera 1 used to describe the focus position. The operation of each component of the dermatoscopy camera 1 is controlled by the control unit 300 on the circuit board 30 shown in FIG. 2. The control unit 300 includes a central processing unit (CPU). The circuit board 30 includes an input-output (I/O) interface, a read-only memory (ROM), and a random-access memory (RAM). The CPU is, for example, a microprocessor, or is a central processing unit that performs various processes and arithmetic operations. The control unit 300 controls the operation of each component of the dermatoscopy camera 1 by reading a control program stored in the ROM and executing the control program on the CPU while using the RAM as a work memory.

The dermatoscopy camera 1 is activated in response to a user operating the power button 22 (FIGS. 1A and 1B) and receives an imaging mode selected by the user (step S11) as shown in FIG. 15. As described above, the imaging mode includes a normal imaging mode for normal imaging, a first dermatoscopy (DC) imaging mode for the first dermatoscopy imaging, and a second DC imaging mode for the second dermatoscopy imaging. The dermatoscopy camera 1 displays three selectable imaging modes on the touchscreen LCD monitor 11 included in the display 10 shown in FIG. 2. The user selects one of the three imaging modes by touching the LCD monitor 11. In response to the selection, the control unit 300 sets an imaging mode to the selected imaging mode.

The control unit 300 determines whether the normal imaging mode is set (step S12). When the normal imaging mode is set in step S12 (Yes in step S12), the control unit 300 determines whether the adapter 70 is attached to the illumination device 4 (step S13). The determination as to whether or not the adapter 70 is attached thereto is made based on the on-off states of the detection switches 62. In other words, the control unit 300 determines that the adapter 70 is detached when the two detection switches 62 protrude from the cover 60 and are in the off state. When the two detection switches 62 are retracted and are in the on state, or when one of the two detection switches 62 is in the on state and the other is in the off state, the control unit 300 determines that the adapter 70 is attached. When one of the detection switches 62 alone is in the on state, the adapter 70 is attached to the cover 60 incompletely.

When the control unit 300 determines that the adapter 70 is detached from the illumination device 4 in step S13 (No in step S13), the LEDs 140 (FIG. 5) for normal imaging are turned on (step S14). This illuminates a diseased skin portion brightly.

When determining, in step S13, that the adapter 70 is attached (Yes in step S13), the control unit 300 indicates that the adapter 70 is to be detached (step S17). For example, the control unit 300 displays a message "Detach the adapter" on the LCD monitor 11 (FIG. 2) to prompt the user to detach the adapter 70. This causes the user to detach the adapter 70 and thus allows the light from the LEDs 140 to illuminate the diseased skin portion brightly during the normal imaging without being blocked by the adapter 70.

Subsequently, in response to the user half-pressing the shutter button 21 shown in FIG. 1A, the control unit 300 moves the lens group in the imaging lens system 41 to automatically focus on the target diseased skin portion within a focus range of P1 to infinity (step S15). The position P1 is a focus start position from which the lens group is moved to infinity to search for a position at which the focus is achieved.

The focus start position P1 will now be described. The two-dot chain lines in FIG. 19 indicate virtual images of the components of the dermatoscopy camera 1 that appear on the rear surface 132b of the first objective cover 132. The virtual images occur symmetrically to the real components about the rear surface 132b. More specifically, a virtual image 41c' of the lens barrel 41c at a distance d1 from the rear surface 132b of the first objective cover 132 occurs oppositely at a distance d1 from the rear surface 132b. Also, virtual images 144' of the LEDs 144 at a distance d2 from the rear surface 132b of the first objective cover 132 occur oppositely at a distance d2 from the rear surface 132b. Similarly, a virtual image 180' of the base body 180 at a distance d3 from the rear surface 132b of the first objective cover 132 occurs oppositely at a distance d3 from the rear surface 132b. When the focus is adjusted to a position within a virtual image range D1 in which the virtual images appear on the rear surface 132b, the distal end of the virtual image 41c' of the lens barrel 41c, the recesses and protrusions and various fixtures on the surface of the virtual image 180' of the base body 180, or the virtual images 144' of the LEDs 144 may be in focus and appear in the captured image. The focus start position P1 is thus set outside the virtual image range D1.

During normal imaging, the user can view the distance between a diseased skin portion as an imaging target and the front surface 132a of the first objective cover 132. Thus, in response to the normal imaging mode set in step S11, the LCD monitor 11 shown in FIG. 2 may display an interval between the diseased skin portion and the first objective cover 132 at which no virtual image appears and prompt the user to adjust the focus position outside the virtual image range D1. This allows the diseased skin portion as an imaging target at a position from the focus start position P1 to infinity, thus allowing autofocus to be smoothly complete.

Subsequently, in response to the user depressing the shutter button 21 shown in FIG. 1A, the control unit 300 captures an image of the diseased skin portion normally and stores the normally captured image (step S16). This completes the normal imaging of the diseased skin portion.

When determining that the first DC imaging mode or the second DC imaging mode is set in step S11 (No in step S12), the control unit 300 performs a dermatoscopy imaging operation (step S20).

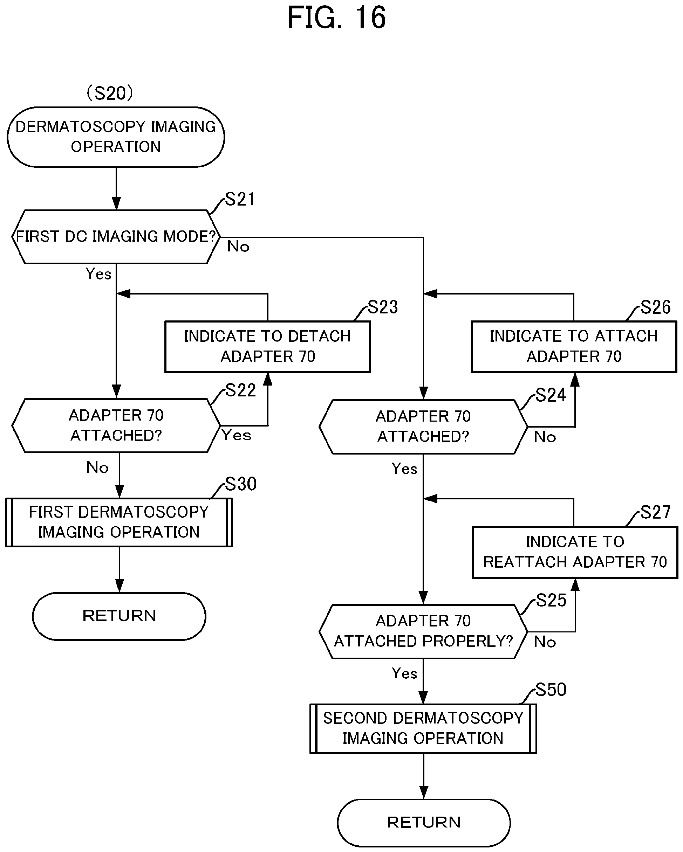

In the dermatoscopy imaging operation (step S20), the control unit 300 first determines whether the first DC imaging mode is set (step S21) as shown in FIG. 16. In the first DC imaging mode (Yes in step S21), the control unit 300 determines whether the adapter 70 is attached to the illumination device 4 (step S22). As described above, the determination as to whether or not the adapter 70 is attached thereto is made based on the on-off states of the detection switches 62. When determining, in step S22, that the adapter 70 is not attached (No in step S22), the control unit 300 performs the first dermatoscopy imaging operation (step S30).

In step S22, when determining that the adapter 70 is attached (Yes in step S22), the control unit 300 indicates that the adapter 70 is to be detached (step S23). This prompts the user to detach the adapter 70, thus allowing the first dermatoscopy imaging operation (S30) to be performed appropriately without the adapter 70 attached.

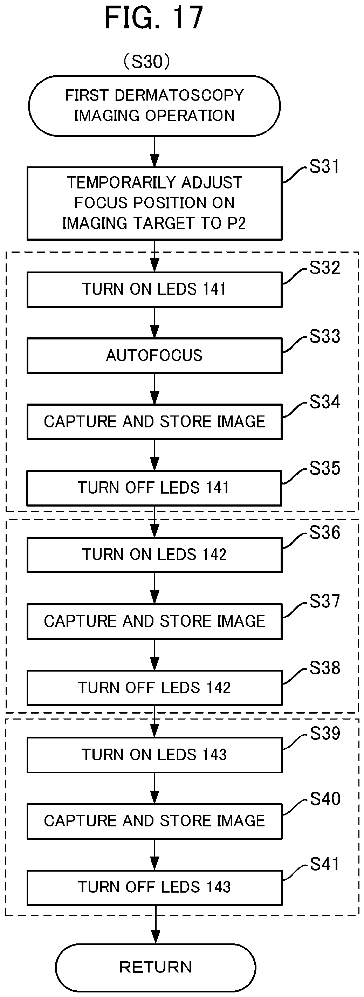

In the first dermatoscopy imaging operation (step S30) shown in FIG. 17, the control unit 300 temporarily adjusts the focus position on the diseased skin portion as an imaging target to P2 (step S31). As shown in FIG. 19, the focus position P2 is aligned with the front surface 132a of the first objective cover 132 that comes in contact with the diseased skin portion. The focus can thus be preadjusted to a position to be aligned with the diseased skin portion as an imaging target.

The control unit 300 then turns on the eight LEDs 141 (FIG. 8) that emit visible light (step S32). This brightly illuminates the diseased skin portion in contact with the first objective cover 132 (FIG. 11) with visible light. Subsequently, in response to the user half-pressing the shutter button 21 shown in FIG. 1A, the control unit 300 automatically focuses on the diseased skin portion as an imaging target (step S33). As described above, the focus position is preadjusted to a position to be aligned with the diseased skin portion that comes in contact with the first objective cover 132 in step S31, thus allowing autofocus to be smoothly complete. Subsequently, in response to the user depressing the shutter button 21 shown in FIG. 1A, the control unit 300 performs the first dermatoscopy imaging of the diseased skin portion and stores the captured image (step S34). The control unit 300 then turns off the eight LEDs 141 (FIG. 8) (step S35). In steps S32 to S35, the first dermatoscopy imaging is performed on the diseased skin portion illuminated with visible light.

The control unit 300 then turns on the eight LEDs 142 (FIG. 8) that emit visible light to emit light polarized by the polarizer plates 117 (FIG. 8) (step S36). This brightly illuminates the diseased skin portion in contact with the first objective cover 132 (FIG. 11) with the polarized light. The control unit 300 then performs the first dermatoscopy imaging of the diseased skin portion and stores the captured image (step S37). The control unit 300 then turns off the eight LEDs 142 (FIG. 8) (step S38). In steps S36 to S38, the first dermatoscopy imaging is performed on the diseased skin portion illuminated with the polarized light.

The control unit 300 then turns on the four LEDs 143 (FIG. 8) that emit ultraviolet light (step S39). This brightly illuminates the diseased skin portion in contact with the first objective cover 132 (FIG. 11) with ultraviolet light. The control unit 300 then performs the first dermatoscopy imaging of the diseased skin portion and stores the captured image (step S40). The control unit 300 then turns off the four LEDs 143 (FIG. 8) (step S41). In steps S39 to S41, the first dermatoscopy imaging is performed on the diseased skin portion illuminated with ultraviolet light.

As described above, in the first dermatoscopy imaging, a single operation on the shutter button 21 (FIG. 1A) enables sequential imaging of the diseased skin portion illuminated with visible light, polarized light, and then ultraviolet light, as well as storing of the captured images. The diseased skin portion is automatically adjusted in focus in step S33, thus eliminating the autofocusing operation in the subsequent processing in the first dermatoscopy imaging.

As shown in FIG. 16, in the second DC imaging mode (No in step S21), the control unit 300 determines whether the adapter 70 is attached to the illumination device 4 (step S24). When at least one of the two detection switches 62 is retracted and in the on state, the control unit 300 determines that the adapter 70 is attached (Yes in step S24), and advances to step S25. When the two detection switches 62 are in the off state (No in step S24), the control unit 300 indicates that the adapter 70 is to be attached (step S26). For example, the control unit 300 displays a message "Attach the adapter" on the LCD monitor 11 (FIG. 2) to prompt the user to attach the adapter 70. This causes the user to attach the adapter 70 and thus allows the second dermatoscopy imaging to be performed appropriately with the adapter 70 attached.

When determining that the adapter 70 is attached (Yes in step S24), the control unit 300 determines whether the adapter 70 is properly attached (step S25). When the two detection switches 62 are in the on state, the control unit 300 determines that the adapter 70 is properly attached (Yes in step S25) and performs a second dermatoscopy imaging operation (step S50).

When determining that the adapter 70 is not properly attached in step S25 (No in step S25), the control unit 300 indicates that the adapter 70 is to be reattached (step S27). For example, the control unit 300 displays, on the LCD monitor 11 (FIG. 2), a message "The adapter is not properly attached. Reattach the adapter. " and prompts the user to reattach the adapter 70. This allows the second dermatoscopy imaging operation (S50) to be performed with the adapter 70 properly attached.

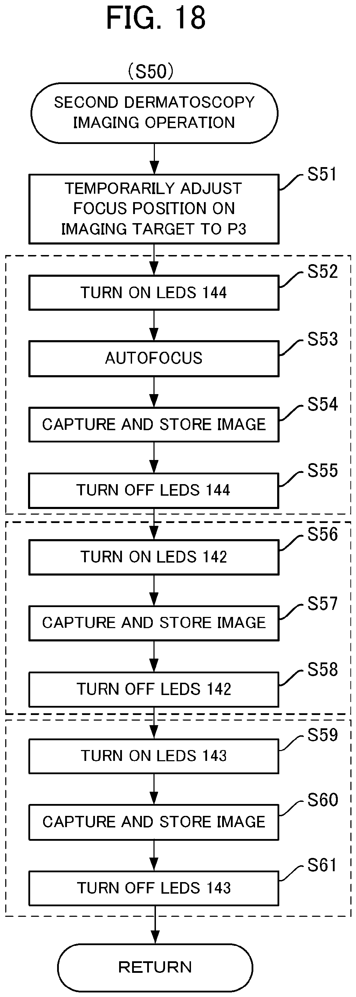

In the second dermatoscopy imaging operation (step S50) shown in FIG. 18, the control unit 300 temporarily adjusts the focus position on the diseased skin portion as an imaging target to P3 (step S51). As shown in FIG. 19, the focus position P3 is aligned with the front surface 72a of the second objective cover 72 that comes in contact with the diseased skin portion during the second dermatoscopy imaging. The focus position can thus be preadjusted to a position to be aligned with the diseased skin portion that comes in contact with the second objective cover 72.

Subsequently, the control unit 300 turns on the four LEDs 144 (FIG. 8) that emit visible light (step S52). The four LEDs 144 are light sources located nearer the optical axis OA for the second dermatoscopy imaging. This brightly illuminates the diseased skin portion in contact with the second objective cover 72 (FIG. 13) with visible light. Subsequently, in response to the user half-pressing the shutter button 21 shown in FIG. 1B, the control unit 300 automatically focuses on the diseased skin portion as an imaging target (step S53). As described above, the focus position is preadjusted to a position to be aligned with the diseased skin portion that comes in contact with the second objective cover 72 in step S34. The control unit 300 thus allows autofocus to be smoothly complete. Subsequently, in response to the user depressing the shutter button 21 shown in FIG. 1B, the control unit 300 performs the second dermatoscopy imaging of the diseased skin portion and stores the captured image (step S54). The control unit 300 then turns off the four LEDs 144 (FIG. 8) (step S55). In steps S52 to S55, the second dermatoscopy imaging is performed on the diseased skin portion illuminated with visible light.

In the subsequent steps S56 to S58, the control unit 300 turns on the LEDs 142 to illuminate the diseased skin portion with polarized light and performs the second dermatoscopy imaging. The processing in steps S56 to S58 is the same as the processing in steps S36 to S38 for the first dermatoscopy imaging shown in FIG. 17, except the use of the adapter 70. In the subsequent steps S59 to S61, the control unit 300 turns on the LEDs 143 to illuminate the diseased skin portion with ultraviolet light and performs the second dermatoscopy imaging. The processing in steps S59 to S61 is the same as the processing in steps S39 to S41 for the first dermatoscopy imaging shown in FIG. 17, except the use of the adapter 70. These steps are thus not described in detail.

As described above, in the second dermatoscopy imaging as well, a single operation on the shutter button 21 (FIG. 1B) enables sequential imaging of the diseased skin portion illuminated with visible light, polarized light, and then ultraviolet light, as well as storing of the captured images. The diseased skin portion is automatically adjusted in focus in step S53, thus eliminating the autofocusing operation in the subsequent processing in the second dermatoscopy imaging.

As described above, the dermatoscopy camera 1 using the illumination device 4 according to one or more embodiments of the present disclosure includes the LEDs 141, 142, and 143 for the first dermatoscopy imaging arranged outward from the first inverted region 501b and within the second inverted region 502b. The LEDs 144 for the second dermatoscopy imaging are arranged outward from the third inverted region 503b and within the fourth inverted region 504b. This prevents the central area of the captured image from being affected by light from the LEDs 141 to 144 reflected by the first objective cover 132 and reduces the likelihood of imaging being affected by the reflected light, while avoiding upsizing the devices.

The LEDs 144 for the second dermatoscopy imaging are arranged outward from the third inverted region 503b that is formed by inverting, with respect to the symmetry axis being the line along the front surface 72a, the third region 503a located between the imaginary upper ray 175a and the imaginary lower ray 178a being the outermost upper and lower rays in the pencil of rays that enters the image sensor 44, or more specifically, outward from the third inverted region 503b located between the inverted upper ray 175' and the inverted lower ray 178'. This arrangement prevents the central area of a captured image from being affected by light from the LEDs 144 arranged in this manner reflected by the second objective cover 72 and reduces the likelihood of imaging being affected by the reflected light.

The LEDs 144 are arranged within the fourth inverted region 504b that is formed by inverting, with respect to the symmetry axis being the line along the front surface 72a of the second objective cover 72, the fourth region 504a located between the imaginary lower ray 176a and the imaginary upper ray 177a, or more specifically, within the fourth inverted region 504b located between the inverted lower ray 176' and the inverted upper ray 177'. The imaginary lower ray 176a and the imaginary upper ray 177a are the outermost rays located at the outermost position among the rays entering the image sensor 44 from the imaging target. The LEDs 144 are arranged within the region defined by these outermost rays to avoid upsizing the devices.

The LEDs 141, 142, and 143 are located away from the optical axis OA. The LEDs 141, 142, and 143 arranged in this manner are angled to face the first objective cover 132 and emit light directed toward the first objective cover 132, thus illuminating the imaging target brightly without unevenness.

The LEDs 144 are separately arranged nearer the optical axis OA for the second dermatoscopy imaging that uses the adapter 70 including the second objective cover 72 with a smaller area. The LEDs 144 nearer the optical axis OA facilitate illumination of the second objective cover 72, thus allowing the imaging target to be illuminated brightly without unevenness.

The base body 180 and the first cover body 121 defining the housing space A for the first LED board 111 provided with the LEDs 140 are formed from a black synthetic resin. The first cover body 121 covers the end faces 111a of the first LED board 111. In other words, the components (the annular portion 182 of the base body 180 and the inner wall 121c) that define the housing space A for the light sources for normal imaging (LEDs 140) and are in the space between the first objective cover 132 (second objective cover 72) and the imager 40 are formed from a material having higher light absorptivity than the other components (e.g., first LED board 111). The base body 180 and the first cover body 121 thus absorb light transmitted through the first LED board 111, preventing the components (e.g., LEDs 144) of the dermatoscopy camera 1 from being illuminated with such light and from appearing in a captured image.

The three types of light sources for dermatoscopy imaging, including the light source that emits visible light, the light source that emits polarized light, and the light source that emits ultraviolet light, allow the diseased skin portion to be illuminated with various rays, thus enabling capturing of images that are useful in diagnosing the skin conditions. Additionally, the diseased skin portion illuminated with visible light, polarized light, and then the ultraviolet light can be imaged in sequence, and the captured images are stored, thus reducing the operation time.

The focus position on the diseased skin portion as an imaging target is temporarily set in accordance with the dermatoscopy imaging mode selected by the user through the LCD monitor 11, or more specifically, in accordance with the imaging mode selected from the first DC imaging mode and the second DC imaging mode in the present embodiment. This temporary focus position is set based on the expected position of the diseased skin portion to be actually imaged, thus allowing quick focus adjustment during actual imaging.

When the normal imaging mode is selected by the user, the focus start position for the diseased skin portion is set outside the virtual image range D1 in which virtual images occur due to the components of the dermatoscopy camera 1 appearing on the first objective cover 132. This prevents the virtual images of the distal end of the lens barrel 41c, the protrusions and recesses and various fixtures on the surface of the base body 180, or the LEDs 144 from appearing in the captured images, and allows proper imaging of the diseased skin portion.

The two detection switches 62 for detecting the adapter 70 are located on the outer circumferential surface of the cover 60 to allow the control unit 300 to determine whether the adapter 70 is attached in each imaging operation. This prevents failures to attach the adapter 70 in the second dermatoscopy imaging to be performed with the adapter 70 attached and failures to detach the adapter 70 in the normal imaging and the first dermatoscopy imaging to be performed without the adapter 70 attached.

The two detection switches 62 are vertically away from each other with the optical axis OA between the switches 62 when the illumination device 4 is viewed from the front. The detection switches 62 away from each other in this manner have, for example, one of the detection switches 62 alone in the on state when the adapter 70 is attached incompletely. This facilitates the control unit 300 to determine whether the adapter 70 is properly attached.

### Embodiment 2

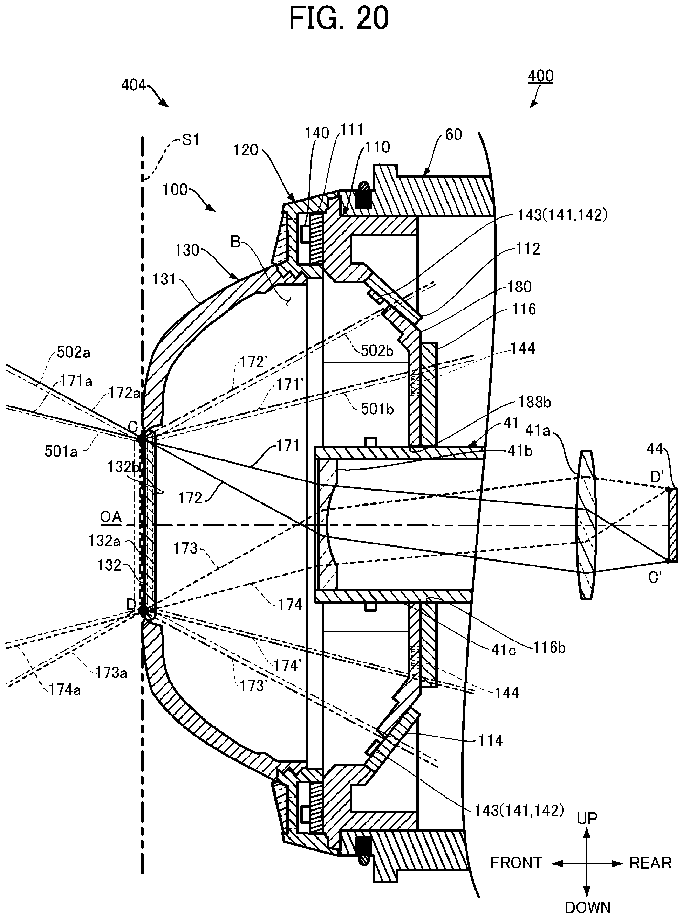

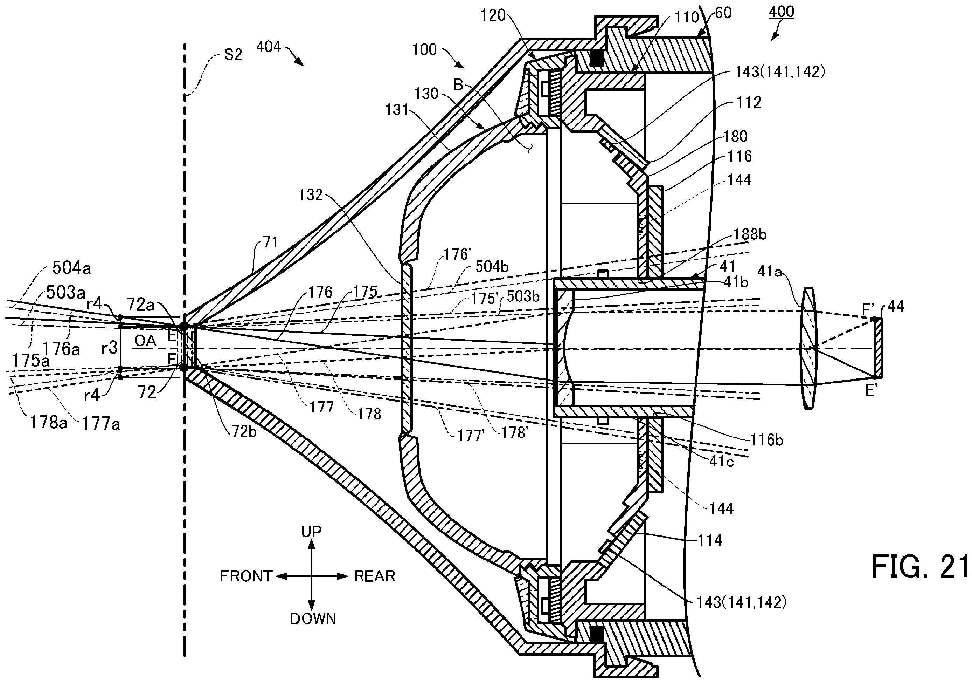

An illumination device according to Embodiment 2 and an imaging device including the illumination device will now be described with reference to FIGS. 20 and 21. The structure in Embodiment 1 reduces the likelihood of imaging being affected by reflected light of the LEDs 141, 142, and 143 used in the first dermatoscopy imaging, while avoiding upsizing the illumination device 4 and the dermatoscopy camera 1. The LEDs 144 used in the second dermatoscopy imaging are located nearer the optical axis OA to facilitate illumination of the second objective cover 72 and reduce the reflected light of the LEDs 144 appearing in the captured image. The structure in Embodiment 2 further reduces the likelihood of (or prevents) imaging being affected by the reflected light. The structure in Embodiment 2 is basically the same as the structure in Embodiment 1 except the dimensions of the components. The components of a dermatoscopy camera 400 according to Embodiment 2 are thus given the same reference numerals as in Embodiment 1.

As shown in FIG. 20, the dermatoscopy camera 400 has the LEDs 141, 142, and 143 for emitting light during the first dermatoscopy imaging located more outward than in Embodiment 1. The LEDs 141, 142, and 143 on the second LED board 112 and the fourth LED board 114 arranged within the second inverted region 502b in Embodiment 1 are arranged outward from the second inverted region 502b in the present embodiment. The LEDs 141, 142, and 143 are arranged in the same manner as in a cross-sectional view taken along a lateral line, or more specifically, the LEDs 141, 142, and 143 on the third LED board 113 (FIG. 8) and the fifth LED board 115 (FIG. 8) are arranged outward from the second inverted region 502b. This prevents imaging from being affected by reflected light of the LEDs 141, 142, and 143 used in the first dermatoscopy imaging.

As shown in FIG. 21, the dermatoscopy camera 400 has the LEDs 144 for emitting light during the second dermatoscopy imaging located more outward than in Embodiment 1. The LEDs 144 on the sixth LED board 116 are outward from the fourth inverted region 504b. The LEDs 144 on the sixth LED board 116 are arranged in the same manner as in a cross-sectional view taken along a lateral line and located outward from the fourth inverted region 504b. This arrangement prevents a captured image from being affected by reflected light of the LEDs 144.

Although the devices are larger in Embodiment 2 than in Embodiment 1, the structure in Embodiment 2 prevents a captured image from being affected by reflected light of the LEDs 141 to 144 and reduces the likelihood of the captured image being affected by stray light.

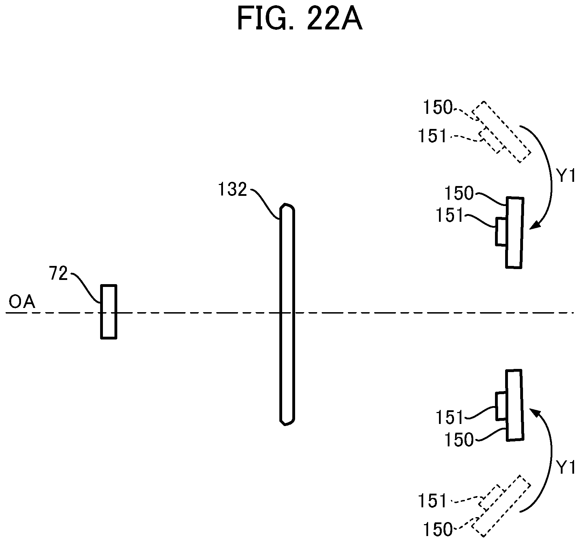

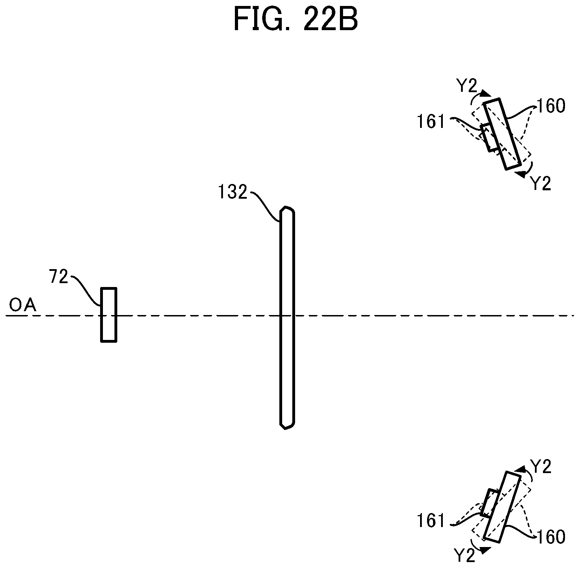

The present disclosure is not limited to the above embodiments and can be modified and altered variously. In the above embodiments, the LEDs 141 that emit visible light during the first dermatoscopy imaging are arranged separately from the LEDs 144 that also emit visible light during the second dermatoscopy imaging. However, in an example not covered by the claims, as shown in FIGS. 22A and 22B, the first dermatoscopy imaging and the second dermatoscopy imaging may commonly use the same light sources that can change the positions or orientations. The LED boards 150 and 160 and the LEDs 151 and 161 indicated by the dashed lines in FIGS. 22A and 22B are at positions during the first dermatoscopy imaging, and the LED boards 150 and 160 and the LEDs 151 and 161 indicated by the solid lines are at positions during the second dermatoscopy imaging.

As shown in FIG. 22A, during the first dermatoscopy imaging, the LED boards 150 (dashed line) are positioned outward, and the LEDs 151 (dashed line) are angled to face the first objective cover 132. For the second dermatoscopy imaging, as indicated by arrows Y1, the LED boards 150 are moved inward by drive means (not shown) while changing the postures to allow the LEDs 151 to face frontward. During the second dermatoscopy imaging, the LED boards 150 (solid line) are thus nearer the optical axis OA, with the LEDs 151 (solid line) facing frontward. The LED boards 150 with the LEDs 151 mounted thereon are moved in the above manner to allow the LEDs 151 to serve as light sources for the first dermatoscopy imaging and for the second dermatoscopy imaging.

As shown in FIG. 22B, during the first dermatoscopy imaging, the LED boards 160 (dashed line) are positioned outward and the LEDs 161 (dashed line) are angled to face the first objective cover 132. For the second dermatoscopy imaging, as indicated by arrows Y2, the LED boards 160 are rotated by drive means (not shown) to have the LEDs 161 facing the second objective cover 72. The LEDs 161 facing the second objective cover 72 in the above manner illuminate the second objective cover 72 entirely, thus illuminating the diseased skin portion in contact with the second objective cover 72 brightly without unevenness. In the manner described above, the LED boards 160 with the LEDs 161 mounted thereon are rotated to allow the LEDs 161 to serve as light sources for the first dermatoscopy imaging and for the second dermatoscopy imaging.

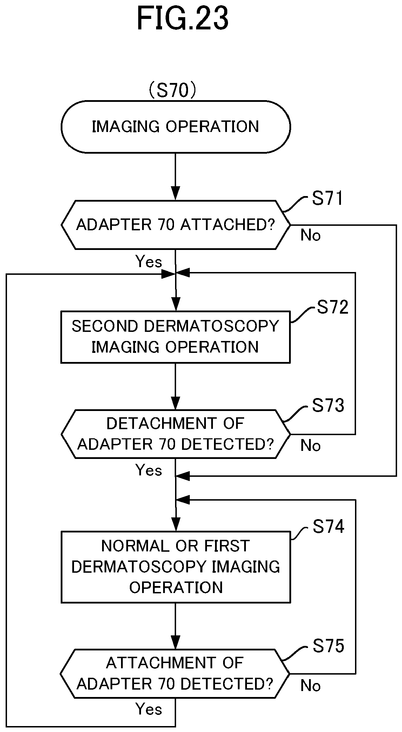

In the above embodiments, the control unit 300 determines whether the adapter 70 is properly attached, and then performs imaging based on the imaging mode input by the user. In other words, the imaging mode is selected basically through an input from the user. The imaging mode may be selected differently. For example, the control unit 300 may determine whether the adapter 70 is attached or detached and then determine the imaging mode without any input from the user about the imaging mode. Such an imaging operation will be described with reference to FIG. 23.

In the imaging operation (step S70), the control unit 300 first determines whether the adapter 70 is attached to the illumination device 4 (step S71). As described above, the determination as to whether or not the adapter 70 is attached thereto is made based on the on-off states of the detection switches 62. In step S71, when determining that the adapter 70 is attached (Yes in step S71), the control unit 300 enters the second DC imaging mode and performs the second dermatoscopy imaging operation in response to an imaging operation performed by the user (step S72). The second dermatoscopy imaging operation (step S72) is the same as the second dermatoscopy imaging operation (S50) shown in FIG. 18. The control unit 300 may display, on the LCD monitor 11 shown in FIG. 2, an indication that the imaging mode is the DC imaging mode before advancing to step S72. This informs the user of the current imaging mode of the camera.

When detecting detachment of the adapter 70 (Yes in step S73) after the second dermatoscopy imaging operation (step S72), the control unit 300 switches to the normal imaging mode or the first DC imaging mode. The control unit 300 then performs the normal imaging operation or the first dermatoscopy imaging operation in accordance with an imaging operation performed by the user (step S74). As described above, in response to detecting detachment of the adapter 70, the control unit 300 switches from the second DC imaging mode to the normal imaging mode or the first DC imaging mode without any operation input by the user. In step S74, the user may select the normal imaging mode or the first DC imaging mode through the LCD monitor 11 shown in FIG. 2.

When detecting attachment of the adapter 70 (Yes in step S75) after the normal imaging operation or the first dermatoscopy imaging operation (step S74), the control unit 300 advances to step S72 and switches the imaging mode to the second DC imaging mode. As described above, in response to detecting attachment of the adapter 70, the control unit 300 switches from the normal imaging mode or the first DC imaging mode to the second DC imaging mode without any operation input by the user.

When the control unit 300 does not detect detachment of the adapter 70 in step S73 (No in step S73), the control unit 300 remains in the second DC imaging mode without switching the imaging mode and performs the second dermatoscopy imaging operation in response to an imaging operation performed by the user (step S72). When the control unit 300 does not detect attachment of the adapter 70 in step S75 (No in step S75), the control unit 300 remains in the normal imaging mode or the first DC imaging mode without switching the imaging mode.

When the control unit 300 determines, in step S71, that the adapter 70 is not attached (No in step S71), the control unit 300 switches to the normal imaging mode or the first DC imaging mode and performs the normal imaging operation or the first dermatoscopy imaging operation in accordance with an imaging operation performed by the user (step S74).

After the normal imaging operation or the first dermatoscopy imaging operation (step S74), the control unit 300 advances to step S71. When determining that the adapter 70 is not attached (Yes in step S71), the control unit 300 switches to the second DC imaging mode. As described above, in response to detecting attachment or detachment of the adapter 70, the control unit 300 switches the imaging mode between the second dermatoscopy imaging operation and the normal imaging operation or the first dermatoscopy imaging operation without any user operation.

In the above embodiments, although the two detection switches 62 are used to detect the adapter 70, any number of detection switches 62, for example more than two detection switches 62, may be used. More than two detection switches 62 allow more accurate determination on whether the adapter 70 is attached. The use of a single detection switch 62 can simplify the structure.

In the above embodiments, when the imaging mode being set does not correctly correspond to the state of the adapter 70 being attached or detached, the LCD monitor 11 shown in FIG. 2 displays the incorrectness. However, other indication means may inform the user of such an error. In one example, the dermatoscopy camera 1 may include a speaker that outputs a sound and produces a beep or a voice to inform the user of an error. In another example, an operation on the shutter button 21 or imaging may be disabled when the imaging mode being set does not correctly correspond to the state of the adapter 70. The user may be informed of an incorrect operation on the shutter button 21 by the LCD monitor 11 displaying a message or by the speaker producing a beep or a voice.

An incorrect correspondence between the imaging mode being set and the state of the adapter 70 being attached or detached may not be indicated in all the situations described above. The indication steps may be eliminated as appropriate. For example, the indication steps S21 to S23 may be eliminated in the first DC imaging mode shown in FIG. 16, or the indication steps S24 to S27 may be eliminated in the second DC imaging mode. The steps S25 and S27 for indicating an incorrect attachment of the adapter 70 may also be eliminated.

Although the detection switches 62 that are turned on and off in response to physical pressure applied by the adapter 70 are used as means for detecting the adapter 70, other detection means may be used. For example, the adapter 70 and the cover 60 include electrical contacts that come in contact with each other and pass a current when the adapter 70 is attached. The detection of the flowing current allows determination on whether the adapter 70 is attached.

In the above embodiments, the light sources are arranged based on the inverted regions formed by inverting, with respect to the objective cover as the symmetry axis, the regions defined by the upper and lower rays from the skin as an imaging target. However, the light sources at a distance from the optical axis OA are less likely to appear in a captured image, thus permitting slight displacement of the symmetry axis about which the inverted region is formed. The inverted region and the region not inverted may not be exactly the same and may be slightly different in shape or dimensions.

The LEDs 141, 142, and 143 may be arranged outward from the second inverted region 502b or arranged, for example, outward from an inverted region defined by imaginary rays using the angle of view of the imager 40 during the first dermatoscopy imaging. The LEDs 144 may also be arranged outward from the fourth inverted region 504b or arranged, for example, outward from an inverted region defined by imaginary rays using the angle of view of the imager 40 during the second dermatoscopy imaging. This arrangement also reduces the likelihood of a captured image being affected by reflected light of the LEDs 141 to 144.

In the embodiments described above, the first to fourth regions 501a to 504a and the first to fourth inverted regions 501b to 504b are truncated conical. However, depending on the aperture in the lens group, these regions may be elliptical truncated conical, rather than being circular truncated conical. For an elliptical truncated conical region, the rays are defined using the major and minor axes of the ellipse to position the light sources.

The illumination device used in the dermatoscopy camera 1 in the above embodiment may also be used in other imaging devices. The illumination device according to one or more embodiments of the present disclosure can be used in any imaging device that illuminates and images an imaging target on one end nearer a light transmissive cover with an imager and a light source on the other end. For example, the illumination device may be used in an imaging device that captures images for inspection of a structure surface or in an imaging device that performs imaging while illuminating an area with a gap into which the imaging device is inserted.

Although the three types of light sources for dermatoscopy imaging include a light source that emits visible light, a light source that emits polarized light, and a light source that emits ultraviolet light, the number of different light sources can be specified as appropriate in accordance with an imaging target. In one example, the illumination device may simply include one of these types of light sources. In another example, the illumination device may include more different types of light sources including, for example, a light source that emits near infrared light.

Although the LEDs 144 that emit visible light for the second dermatoscopy imaging using the adapter 70 are located nearer the optical axis OA, the illumination device may or may not include such a light source for the second dermatoscopy imaging. Other types of light sources may be located nearer the optical AX for the second dermatoscopy imaging, such as a light source that emits polarized light and a light source that emits ultraviolet light. Thus, the light sources for the first dermatoscopy imaging may include all the different types of light sources, and the light sources for the second dermatoscopy imaging may also include all the different light sources.

The second cover 130 with the first objective cover 132 is attachable and detachable with screws, and the adapter 70 with the second objective cover 72 is attachable and detachable with a hooked engagement member. However, the imaging device may include other attachment and detachment means. For example, the second cover 130 and the adapter 70 may pivot on a pivot axis between the position at which the front of the imaging device is covered and the position at which the front of the imaging device is uncovered.

For the second dermatoscopy imaging, although the adapter 70 is attached with the second cover 130 attached as shown in FIG. 6, the second cover 130 may be detached before the adapter 70 is attached. This eliminates the second cover 130 for the first dermatoscopy imaging located between the adapter 70 and the imager 40 during the second dermatoscopy imaging, thus facilitating the design of the dermatoscopy camera 1 suitable for the second dermatoscopy imaging. To have the second cover 130 detached reliably for the second dermatoscopy imaging, the second cover 130 may have a larger outer diameter or a protrusion to prevent the adapter 70 from being attached over the second cover 130. The adapter 70 may have external threads on the bottom (rear end) to be engaged with the internal threads 121e (FIG. 9) on the inner wall 121c.

The second cover 130 with the first objective cover 132 may not be detachable, and may be fixed to, for example, the first cover 120 (FIG. 5). In another example, the second cover 130 may be integral with the first cover 120 (FIG. 5). This simplifies the structure of the dermatoscopy camera 1.

When the focus position on the imaging target is in the virtual image range D1 during the normal imaging, an operation on the shutter button 21 or imaging may be disabled. In this case, the LCD monitor 11 may display an image that prompts moving the dermatoscopy camera 1 further away from the imaging target. This allows the user to image the imaging target from an appropriate position without causing a virtual image to appear.

Although a single operation on the shutter button 21 enables imaging to be repeated using different types of light sources emitting light in sequence during dermatoscopy imaging, one or more light sources specified by the user may be used to emit light for dermatoscopy imaging.

The adapter 70 for imaging narrow areas is optional. An imaging device that is not designed for imaging narrow areas can eliminate the adapter 70. Such an imaging device not designed for the second dermatoscopy imaging can eliminate the light source for the second dermatoscopy imaging (e.g., LEDs 144).

Although the base body 180 and the first cover body 121 defining the housing space A for the first LED board 111 are formed from, for example, a black synthetic resin to avoid illuminating the interior of the dermatoscopy camera 1 with light from the light sources for normal imaging (LEDs 140), the base body 180 and the first cover body 121 may be formed from a synthetic resin of another color that easily absorbs light. To reduce light leakage to the interior of the dermatoscopy camera 1, the base body 180 and the first cover body 121 separating the space A from the space B shown in FIG. 9 may include other structures that appropriately block light from the light sources located in the space A for normal imaging.

For example, as shown in FIG. 9, the outer surface A1 of the first cover body 121 and the outer surface A2 of the base body 180 may be mirror-finished to reflect light L1 and light L2, thus preventing light from entering the first cover body 121 and the base body 180. For mirror-finishing, known mirror-finishing techniques can be used, such as a silver film or chrome plating applied to the surface. As described above, the first cover body 121 and the base body 180 including light reflection means with higher light reflectivity may reduce light leakage to the interior of the dermatoscopy camera 1.

In another example, a commercially available masking tape, rubber plate, or metal plate may be attached to the outer surface A1 of the first cover body 121 and the outer surface A2 of the base body 180 to block light from the light sources (LEDs 140).

In still another example, the first cover body 121 and the base body 180 may be formed from a lightproof material, for example, from a metal such as aluminum or stainless steel, or from a black rubber material.

As described above, the base body 180 and the first cover body 121 separating the housing space A from the space B include light blocking means for blocking light to reduce leakage of light from the LEDs 140 located in the housing space A for normal imaging to the distal end of the lens barrel 41c or to the space B housing the LEDs 141, 142, 143, and 144 for dermatoscopy imaging. Various light blocking means described above may be used.

### Industrial Applicability

The technique according to one or more embodiments of the present disclosure is particularly useful for allowing proper imaging of a target by reducing the likelihood of imaging being affected by reflected light of a light source.

### Reference Signs List

- 1: Dermatoscopy camera
- 2: Controller
- 3: Camera body
- 4: Illumination device
- 10: Display
- 11: LCD monitor
- 20: Body
- 21: Shutter button
- 22: Power button
- 30: Circuit board
- 40: imager
- 41: Imaging lens system
- 41a: First imaging lens
- 41b: Second imaging lens
- 41c: Lens barrel
- 43: Wiring circuit board
- 44: Image sensor
- 45: Infrared cut filter
- 46: Ultraviolet transmissive filter
- 47: Polarization filter
- 48: Flexible circuit board
- 50: Frame
- 60: Cover
- 61: Protrusion
- 62: Detection switch
- 62a: Spring
- 70: Adapter
- 70a: Inner wall
- 71: Cylinder
- 71a: Opening
- 71b: Hooked portion
- 72: Second objective cover
- 72a: Front surface
- 73, 74: Second objective cover area
- 74a: Darker area
- 100: Illumination device body
- 110: Base
- 111 to 116: First to sixth LED boards
- 116a: Barrel insertion hole
- 117: Polarizer plate
- 120: First cover
- 121: First cover body
- 121a: Cover portion
- 121b: Outer wall
- 121c: Inner wall
- 121d: light releasing hole
- 121e: Internal thread
- 122: Translucent plate
- 130: Second cover
- 131: Cylinder
- 131a: Opening
- 131b: External thread
- 132: First objective cover
- 132a: Front surface
- 132b: Rear surface
- 140 to 144: LED
- 150: LED board
- 151: LED
- 160: LED board
- 161: LED
- 171, 173, 175, 177: Upper ray
- 171', 173', 175', 177': Inverted upper ray
- 172, 174, 176, 178: Lower ray
- 172', 174', 176', 178': Inverted lower ray
- 180: Base body
- 181: Cylindrical portion
- 182: Annular portion
- 182: Board mounting surface
- 183 to 186: First to Fourth walls
- 187: Connection part
- 188: Fifth wall
- 188b: Barrel insertion hole
- 189: Screw
- 200: Storage
- 300: Control unit
- 400: Dermatoscopy camera
- 404: Illumination device
- 501a: First region
- 501b: First inverted region
- 502a: Second region
- 502b: Second inverted region
- 503a: Third region
- 503b: Third inverted region
- 504a: Fourth region
- 504b: Fourth inverted region
- A: Housing space
- D1: Virtual image range
- P1: Focus start position
- P2, P3: Focus position
- S1, S2: Skin
- OA: Optical axis
- r1 to r4: Region

## Claims

1. An imaging device (1) for imaging a human body as a target, the imaging device (1) comprising:
an illumination device (4) suitable for use with an imager (40) for imaging a target, and
the imager (40), wherein the illumination device (4) comprising:
an objective cover (72, 132) including a light transmissive member, the objective cover (72, 132) being configured to direct light from the target to the imager (40) to allow the imager (40) to image the target; and
at least one light source (141, 142, 143, 144) located nearer the imager (40) than the objective cover (72, 132) to illuminate the target through the objective cover (72, 132),
wherein the imager (40) includes an image sensor (44) configured to image the target and an imaging lens (41a, 41b) configured to form an image of the target on the image sensor (44), and
the at least one light source (141, 142, 143, 144) is located outward from an inverted region that is a region formed by substantially inverting, toward the imager (40) and with respect to the objective cover (72,132), a region defined by predetermined rays of imaginary rays nearer the target from the objective cover (72,132) in a pencil of rays, the pencil of rays entering, through the imaging lens (41a, 41b), the image sensor (44) from the target in contact with the objective cover (72, 132) and in focus with the imaging lens (41a, 41b),
wherein the objective cover (72, 132) includes a first objective cover (132) and a second objective cover (72), and at least one of the first objective cover (132) or the second objective cover (72) is selectively usable,
the first objective cover (132) has a predetermined first area being an area of a surface orthogonal to an optical axis of the imager (40),
the second objective cover (72) has a predetermined second area being an area of a surface orthogonal to the optical axis of the imager (40), and the second area is smaller than the first area,
the at least one light source includes at least one first light source (141, 142, 143) to illuminate the target through the first objective cover (132) and at least one second light source (144) nearer to the optical axis to illuminate the target through the second objective cover (72),
the at least one first light source (141, 142, 143) is located outward from a first inverted region that is a region formed by substantially inverting, toward the imager (40) and with respect to the first objective cover (132), a region defined by first predetermined rays of imaginary rays nearer the target from the first objective cover (132) in a pencil of rays, the pencil of rays entering, through the imaging lens (41a, 41b), the image sensor (44) from the target in contact with the first objective cover (132) and in focus with the imaging lens (41a, 41b), and
the at least one second light source (144) is located outward from a second inverted region that is a region formed by substantially inverting, toward the imager (40) and with respect to the second objective cover (72), a region defined by second predetermined rays of imaginary rays nearer the target from the second objective cover (72) in a pencil of rays, the pencil of rays entering, through the imaging lens (41a, 41b), the image sensor (44) from the target in contact with the second objective cover (72) and in focus with the imaging lens (41a, 41b)..

2. The imaging device (1) according to claim 1, wherein
among the rays entering the image sensor (44), the predetermined rays include an upper ray (171a, 175a) located at an outermost position among a plurality of upper rays included in the pencil of rays and a lower ray (174a, 178a) located at an outermost position among a plurality of lower rays included in the pencil of rays.

3. The imaging device (1) according to claim 1, wherein
among the rays entering the image sensor (44), the predetermined rays include outermost rays (172a, 173a, 176a, 177a) located at outermost positions among a plurality of rays included in the pencil of rays.

4. The imaging device (1) according to any one of claims 1 to 3, wherein
the inverted region is a region formed by substantially inverting the region defined by the predetermined rays toward the imager (40) and with respect to a surface of the objective cover (72, 132) adjacent to the target.

5. The imaging device (1) according to any one of claims 1 to 4, wherein
the at least one light source includes a plurality of light sources (141, 142, 143) arranged symmetrical to one another about an optical axis of the imager (40) in a direction orthogonal to the optical axis of the imager (40).

6. The imaging device (1) according to claim 5, wherein
each of the plurality of light sources (141, 142, 143) is located to emit light directed toward the objective cover (72, 132) to focus on an overall area of the objective cover (72, 132).

7. The imaging device (1) according to claim 5 or 6, **characterized in that**
the plurality of light sources (141,142,143) include at least one type of light source selected from the group consisting of a light source to emit visible light, a light source to emit ultraviolet light, and a light source to emit polarized light of visible light.

8. The imaging device (1)according to claim 1, wherein
the first imaginary rays include an upper ray (171a) located at an outermost position among a plurality of upper rays included in the pencil of rays and a lower ray (174a) located at an outermost position among a plurality of lower rays included in the pencil of rays, the pencils of rays entering, through the imaging lens (41a, 41b), the image sensor (44) from the target in contact with the first objective cover (132) and in focus with the imaging lens (41a, 41b) and
the second imaginary rays include an upper ray (175a) located at an outermost position among a plurality of upper rays included in the pencil of rays and a lower ray (178a) located at an outermost position among a plurality of lower rays included in the pencil of rays, the pencil of rays entering, through the imaging lens (41a, 41b), the image sensor (44) from the target in contact with the second objective cover (72) and in focus with the imaging lens (41a, 41b).

9. The imaging device (1) according to claim 1, wherein
the first predetermined rays include outermost rays (172a, 173a) located at outermost positions among a plurality of imaginary rays included in the pencil of rays, the pencil of rays entering through the imaging lens (41a, 41b), the image sensor (44) from the target in contact with the first objective cover (132) and in focus with the imaging lens (41a, 41b), and
the second predetermined rays include outermost rays (176a, 177a) located at outermost positions among a plurality of imaginary rays included in the pencil of rays, the pencil of rays entering through the imaging lens (41a,41b), the image sensor (44) from the target in contact with the second objective cover (72) and in focus with the imaging lens (41a,41b).

10. The imaging device (1) according to any one of claims 1 to 9, wherein
the first inverted region is a region formed by substantially inverting the region defined by the first predetermined rays toward the imager (40) and with respect to a surface of the objective cover (132) adjacent to the target, and
the second inverted region is a region formed by substantially inverting the region defined by the second predetermined rays toward the imager (40) and with respect to the surface of the objective cover (72) adjacent to the target.

11. The imaging device (1) according to any one of claims 1 to 10, wherein
the at least one first light source (141, 142, 143) includes a plurality of first light sources each located to emit light directed toward the first objective cover (132) and focus on an overall area of the first objective cover (132), and
the at least one second light source (144) includes a plurality of second light sources each located to emit light directed toward the second objective cover (72) and focus on an overall area of the second objective cover (72).

12. The imaging device (1) according to claim 11, wherein
the plurality of first light sources and the plurality of second light sources include common light sources (151, 161), and
the common light sources (151, 161) selectively function as the plurality of first light sources or the plurality of second light sources by changing positions of the common light sources (151, 161) relative to the first objective cover (132) or the second objective cover (72) or changing an angle of light emitted from each of the common light sources (151, 161).

13. The imaging device (1) according to any one of claims 1 to 12, wherein
the at least one first light source includes at least one type of light source selected from the group consisting of a light source for visible light, a light source for ultraviolet light, and a light source for polarized light of visible light.

14. The imaging device (1) according to any one of claims 1 to 13, wherein the objective cover (72, 132) included in the illumination device (4) comes in contact with the skin to be imaged with the imaging device (1).

15. The imaging device (1) according to any one of claims 1 to 13; wherein the first objective cover (132) is at a top of a first cover (131) included in the illumination device (4), and the first cover (131) is cylindrical,
the second objective cover (72) is at a top of a second cover (71) being cylindrical,
the second cover (71) includes, in a portion other than the top, attachment and detachment means (71b) for attaching and detaching the second cover (71) to and from the illumination device (4),
the first objective cover (132) is used as the objective cover to be in contact with the skin, with the second cover (71) detached from the illumination device (4) with the attachment and detachment means (71b), and
the second objective cover (72) is used as the objective cover to be in contact with the skin, with the second cover (71) attached to the illumination device (4) with the attachment and detachment means (71b) and covering the first cover (131).

16. The imaging device (1) according to claim 15, further comprising:
setting means (11) for selectively setting, in accordance with an operation performed by a user of the imaging device (1), an imaging mode to image the target with the imager (40) to one of a plurality of imaging modes including a first imaging mode to image the target illuminated by the at least one first light source (141, 142, 143) without the at least one second light source (144) and a second imaging mode to image the target illuminated by the at least one second light source (144) without the at least one first light source (141, 142, 143);
detection means (62) for detecting the second cover (71) in a state attached or detached to or from the illumination device (4); and
indication means (11) for informing, in response to at least one of conditions being satisfied, the user that the at least one condition is satisfied, the conditions including the detection means (62) detecting the second cover (71) attached to the illumination device (4) and the imaging mode set to the first imaging mode, and the detection means (62) detecting the second cover (71) detached from the illumination device (4) and the imaging mode set to the second imaging mode.

17. The imaging device (1) according to claim 15, further comprising:
setting means (11) for selectively setting, in accordance with an operation performed by a user of the imaging device (1), an imaging mode to image the target with the imager (40) to one of a plurality of imaging modes including a first imaging mode to image the target illuminated by the at least one first light source (141, 142, 143) without the at least one second light source (144) and a second imaging mode to image the target illuminated by the at least one second light source (144) without the at least one first light source (141, 142, 143); and
detection means (62) for detecting attachment and detachment of the second cover (71) to and from the illumination device (4),
wherein the setting means (11) switches the imaging mode to the second imaging mode in response to the detection means (62) detecting attachment of the second cover (71) to the illumination device (4) in a state where the imaging mode is set to the first imaging mode, and switches the imaging mode to one of the plurality of imaging modes other than the second imaging mode in response to the detection means (62) detecting detachment of the second cover (71) from the illumination device (4) in a state where the imaging mode is set to the second imaging mode.

## Patentansprüche

1. Abbildungsvorrichtung (1) zum Abbilden eines menschlichen Körpers als Ziel, wobei die Abbildungsvorrichtung (1) aufweist: eine Beleuchtungsvorrichtung (4), die zur Verwendung mit einem Bildgeber (40) zum Abbilden eines Ziels geeignet ist, und den Bildgeber (40), wobei die Beleuchtungsvorrichtung (4) aufweist:
eine Objektivabdeckung (72, 132) mit einem lichtdurchlässigen Element, wobei die Objektivabdeckung (72, 132) so konfiguriert ist, dass sie Licht vom Ziel zum Bildgeber (40) leitet, damit der Bildgeber (40) das Ziel abbilden kann; und
mindestens eine Lichtquelle (141, 142, 143, 144), die näher an dem Bildgeber (40) als an der Objektivabdeckung (72, 132) angeordnet ist, um das Ziel durch die Objektivabdeckung (72, 132) zu beleuchten,
wobei der Bildgeber (40) einen Bildsensor (44) hat, der so konfiguriert ist, dass er das Ziel abbildet, und eine Abbildungslinse (41a, 41b) umfasst, die so konfiguriert ist, dass sie ein Bild des Ziels auf dem Bildsensor (44) erzeugt, und
die mindestens eine Lichtquelle (141, 142, 143, 144) außerhalb eines invertierten Bereichs angeordnet ist, der ein Bereich ist, der durch im Wesentlichen Invertieren geformt ist, zum Bildgeber (40) und mit Bezug auf die Objektivabdeckung (72, 132), ein Bereich definiert durch vorbestimmte Strahlen von imaginären Strahlen näher am Ziel
von der Objektivabdeckung (72, 132) in einem Strahlenbündel, wobei das Strahlenbündel durch die Abbildungslinse (41a, 41b) in den Bildsensor (44) vom Ziel in Kontakt mit der Objektivabdeckung (72, 132) und im Fokus mit der Abbildungslinse (41a, 41b) eintritt,
wobei die Objektivabdeckung (72, 132) eine erste Objektivabdeckung (132) und eine zweite Objektivabdeckung (72) umfasst und mindestens eine der ersten Objektivabdeckung (132) oder der zweiten Objektivabdeckung (72) selektiv verwendbar ist,
die erste Objektivabdeckung (132) einen vorbestimmten ersten Bereich aufweist, der ein Bereich einer Oberfläche orthogonal zu einer optischen Achse des Bildgebers (40) ist,
die zweite Objektivabdeckung (72) eine vorbestimmte zweite Fläche aufweist, die eine Fläche einer Oberfläche orthogonal zu der optischen Achse des Bildgebers (40) ist, und die zweite Fläche kleiner als die erste Fläche ist.
die mindestens eine Lichtquelle mindestens eine erste Lichtquelle (141, 142, 143) hat um
das Ziel durch die erste Objektivabdeckung (132) zu beleuchten, und mindestens eine zweite Lichtquelle (144) näher an der optischen Achse hat, um das Ziel durch die zweite Objektivabdeckung (72) zu beleuchten,
wobei die mindestens eine erste Lichtquelle (141, 142, 143) außerhalb eines ersten invertierten
Bereich, der durch ein im Wesentlichen umgekehrtes, zum Bildgeber (40) und in Bezug auf die erste Objektivabdeckung (132) hin ausgerichtetes Gebiet gebildet wird, das durch erste vorbestimmte Strahlen von imaginären Strahlen näher am Ziel von der ersten Objektivabdeckung (132) in einem Strahlenbündel ist, wobei das Strahlenbündel durch die Abbildungslinse (41a, 41b) in den Bildsensor (44) von dem Ziel in Kontakt mit der ersten Objektivabdeckung (132) und in Fokus mit der Abbildungslinse (41a, 41b) eintritt, und
die mindestens eine zweite Lichtquelle (144) außerhalb eines zweiten invertierten Bereichs angeordnet ist, der ein Bereich ist, der durch im Wesentlichen Umkehren eines Bereichs, der durch zweite vorbestimmte Strahlen von imaginären Strahlen definiert ist, in Richtung des Bildgebers (40) und in Bezug auf die zweite Objektivabdeckung (72) näher am
Ziel von der zweiten Objektivabdeckung (72) in einem Strahlenbündel näher ist, wobei das Strahlenbündel durch die Abbildungslinse (41a, 41b) in den Bildsensor (44) von dem Ziel in Kontakt mit der zweiten Objektivabdeckung (72) und in Fokus mit der Abbildungslinse (41a, 41b) eintritt.

2. Abbildungsvorrichtung (1) nach Anspruch 1, wobei
unter den Strahlen, die in den Bildsensor (44) eintreten, die vorbestimmten Strahlen einen oberen Strahl (171a, 175a), der sich an einer äußersten Position unter einer Vielzahl von oberen Strahlen befindet, die in dem Strahlenbündel enthalten sind, und einen unteren Strahl (174a, 178a), der sich an einer äußersten Position unter einer Vielzahl von unteren Strahlen befindet, die in dem Strahlenbündel enthalten sind, umfassen.

3. Abbildungsvorrichtung (1) nach Anspruch 1, wobei
unter den Strahlen, die in den Bildsensor (44) eintreten, die vorbestimmten Strahlen äußerste Strahlen (172a, 173a, 176a, 177a) umfassen, die sich an äußersten Positionen unter einer Vielzahl von Strahlen befinden, die in dem Strahlenbündel enthalten sind.

4. Abbildungsvorrichtung (1) gemäß einem der Ansprüche 1 bis 3, wobei
der invertierte Bereich ein Bereich ist, der durch im Wesentlichen Invertieren des durch die vorbestimmten Strahlen definierten Bereichs in Richtung des Bildgebers (40) und in Bezug auf eine Oberfläche der Objektivabdeckung (72, 132) neben dem Ziel gebildet wird.

5. Abbildungsvorrichtung (1) gemäß einem der Ansprüche 1 bis 4, wobei
die mindestens eine Lichtquelle eine Vielzahl von Lichtquellen (141, 142, 143) umfasst, die symmetrisch zueinander um eine optische Achse des Bildgebers (40) in einer Richtung orthogonal zu der optischen Achse des Bildgebers (40) angeordnet sind.

6. Abbildungsvorrichtung (1) gemäß Anspruch 5, wobei
jede der mehreren Lichtquellen (141, 142, 143) so angeordnet ist, dass sie Licht emittiert, das auf die Objektivabdeckung (72, 132) gerichtet ist, um auf einen Gesamtbereich der Objektivabdeckung (72, 132) zu fokussieren.

7. Abbildungsvorrichtung (1) gemäß Anspruch 5 oder 6, **dadurch gekennzeichnet, dass**
die Vielzahl von Lichtquellen (141, 142, 143) mindestens einen Typ von Lichtquelle umfasst, der aus der Gruppe ausgewählt ist, die aus einer Lichtquelle zum Emittieren von sichtbarem Licht, einer Lichtquelle zum Emittieren von ultraviolettem Licht und einer Lichtquelle zum Emittieren von polarisiertem Licht von sichtbarem Licht besteht.

8. Abbildungsvorrichtung (1) gemäß Anspruch 1, wobei
die ersten imaginären Strahlen einen oberen Strahl (171a), der sich an einer äußersten Position unter einer Vielzahl von oberen Strahlen befindet, die in dem Strahlenbündel enthalten sind, und einen unteren Strahl (174a) umfassen, der sich an einer äußersten Position unter einer Vielzahl von unteren Strahlen befindet, die in dem Strahlenbündel enthalten sind, wobei die Strahlenbündel durch die Abbildungslinse (41a, 41b) in den Bildsensor (44) ein, und zwar von der Aufgabe, die mit der ersten Objektivabdeckung (132) in Kontakt steht und mit der Abbildungslinse (41a, 41b) fokussiert ist, und
die zweiten imaginären Strahlen umfassen einen oberen Strahl (175a), der sich an einer äußersten Position unter einer Vielzahl von oberen Strahlen, die in dem Strahlenbündel enthalten sind, und einen unteren Strahl (178a), der an einer äußersten Position unter einer Vielzahl von unteren Strahlen angeordnet ist, die in dem Strahlenbündel enthalten sind, wobei das Strahlenbündel durch die Abbildungslinse (41a, 41b) in den Bildsensor (44) von dem Ziel eintritt, das in Kontakt mit der zweiten Objektivabdeckung (72) und in Fokus mit der Abbildungslinse (41a, 41b).

9. Abbildungsvorrichtung (1) nach Anspruch 1, wobei
die ersten vorbestimmten Strahlen äußerste Strahlen (172a, 173a) umfassen, die sich an äußersten Positionen unter einer Vielzahl von imaginären
Strahlen befinden, die in dem Strahlenbündel enthalten sind, wobei das Strahlenbündel
durch die Abbildungslinse (41a, 41b), den Bildsensor (44) von dem Ziel in Kontakt mit der ersten Objektivabdeckung (132) und in Fokus mit der Abbildungslinse (41a, 41b) eintritt, und
die zweiten vorbestimmten Strahlen äußerste Strahlen (176a, 177a) umfassen, die an äußersten
Positionen unter einer Vielzahl von imaginären Strahlenbündel, das durch die Abbildungslinse (41a, 41b) eintritt, wobei der Bildsensor (44) von dem Ziel in Kontakt mit der zweiten Objektivabdeckung (72) und in Fokus mit der Abbildungslinse (41a, 41b) ist.

10. Abbildungsvorrichtung (1) gemäß einem der Ansprüche 1 bis 9, wobei
der erste invertierte Bereich ein Bereich ist, der durch im Wesentlichen Invertieren des Bereichs gebildet wird, der durch die ersten vorbestimmten Strahlen in Richtung des Bildgebers (40) und in Bezug auf eine Oberfläche der Objektivabdeckung (132) benachbart zu dem Ziel definiert ist, und
der zweite invertierte Bereich ein Bereich ist, der durch im Wesentlichen Invertieren des Bereichs gebildet wird, der durch die zweiten vorbestimmten Strahlen in Richtung des Bildgebers (40) und in Bezug auf die Oberfläche der Objektivabdeckung (72) benachbart zu dem Ziel definiert ist.

11. Abbildungsvorrichtung (1) gemäß einem der Ansprüche 1 bis 10, wobei
die mindestens eine erste Lichtquelle (141, 142, 143) eine Vielzahl von ersten Lichtquellen umfasst, die jeweils so angeordnet sind, dass sie Licht emittieren, das auf die erste Objektivabdeckung (132) gerichtet ist und auf einen Gesamtbereich der ersten Objektivabdeckung (132) fokussiert wird, und
die mindestens eine zweite Lichtquelle (144) eine Vielzahl von zweiten Lichtquellen umfasst, die jeweils so angeordnet sind, dass sie Licht emittieren, das auf die zweite Objektivabdeckung (72) gerichtet ist, und auf einen Gesamtbereich der zweiten Objektivabdeckung (72) fokussieren.

12. Abbildungsvorrichtung (1) gemäß Anspruch 11, wobei
die Vielzahl erster Lichtquellen und die Vielzahl zweiter Lichtquellen gemeinsame Lichtquellen (151, 161) umfassen und
die gemeinsamen Lichtquellen (151, 161) selektiv als die Vielzahl von ersten Lichtquellen oder die Vielzahl von zweiten Lichtquellen fungieren, indem Positionen der gemeinsamen Lichtquellen (151, 161) relativ zu der ersten Objektivabdeckung (132) oder der zweiten Objektivabdeckung (72) geändert werden oder ein Winkel von Licht, das von jeder der gemeinsamen Lichtquellen (151, 161) emittiert wird, geändert wird.

13. Abbildungsvorrichtung (1) gemäß einem der Ansprüche 1 bis 12, wobei
die mindestens eine erste Lichtquelle mindestens einen Typ von Lichtquelle umfasst, der aus der Gruppe ausgewählt ist, die aus einer Lichtquelle für sichtbares Licht, einer Lichtquelle für ultraviolettes Licht und einer Lichtquelle für polarisiertes Licht von sichtbarem Licht besteht.

14. Abbildungsvorrichtung (1) gemäß einem der Ansprüche 1 bis 13, wobei die in der Beleuchtungsvorrichtung (4) enthaltene Objektivabdeckung (72, 132) mit der Haut in Kontakt kommt, die mit der Abbildungsvorrichtung (1) abgebildet werden soll.

15. Abbildungsvorrichtung (1) gemäß einem der Ansprüche 1 bis 13, wobei sich die erste Objektivabdeckung (132) an einer Oberseite einer ersten Abdeckung (131) befindet, die in der Beleuchtungsvorrichtung (4) enthalten ist, und die erste Abdeckung (131) zylindrisch ist,
sich die zweite Objektivabdeckung (72) an einer Oberseite einer zweiten Abdeckung (71) befindet, die zylindrisch ist,
die zweite Abdeckung (71) in einem anderen Abschnitt als dem oberen Abschnitt Befestigungs- und Ablösemittel (71b) zum Befestigen und Ablösen der zweiten Abdeckung (71) an der bzw. von der Beleuchtungsvorrichtung (4) umfasst,
die erste Objektivabdeckung (132) als Objektivabdeckung verwendet wird, die mit der Haut in Kontakt kommt, wobei die zweite Abdeckung (71) mit den Befestigungs- und Ablösemitteln (71b) von der Beleuchtungsvorrichtung (4) gelöst ist, und
die zweite Objektivabdeckung (72) als die Objektivabdeckung verwendet wird, die mit der Haut in Kontakt steht, wobei die zweite Abdeckung (71) an der Beleuchtungsvorrichtung (4) mit der Befestigungs- und Ablösemöglichkeit (71b) befestigt ist und die erste Abdeckung (131) abdeckt.

16. Abbildungsvorrichtung (1) gemäß Anspruch 15, die ferner aufweist:
Einstellmittel (11) zum selektiven Einstellen, in Übereinstimmung mit einer von einem Benutzer der Abbildungsvorrichtung (1) ausgeführten Operation, einen Bildgebungsmodus zum Abbilden des Ziels mit dem Bildgeber (40) auf einen von mehreren Bildgebungsmodi, einschließlich eines ersten Bildgebungsmodus zum Abbilden des Ziels, das durch die mindestens eine erste Lichtquelle (141, 142, 143) beleuchtet wird, ohne die mindestens eine zweite Lichtquelle (144), und eines zweiten Bildgebungsmodus zum Abbilden des Ziels, das durch die mindestens eine zweite Lichtquelle (144) beleuchtet wird, ohne die mindestens eine erste Lichtquelle (141, 142, 143);
Erfassungsmittel (62) zum Erfassen der zweiten Abdeckung (71) in einem Zustand, in dem sie an der Beleuchtungsvorrichtung (4) angebracht oder von dieser abgenommen ist; und
Anzeigemittel (11), um den Benutzer in Reaktion auf die Erfüllung mindestens einer der Bedingungen darüber zu informieren, dass die mindestens eine Bedingung erfüllt ist, wobei die Bedingungen Folgendes umfassen: die Erfassungseinrichtung (62) erfasst die zweite Abdeckung (71), die an der Beleuchtungsvorrichtung (4) angebracht ist, und der Bildgebungsmodus ist auf den ersten Bildgebungsmodus eingestellt, und die Erfassungseinrichtung (62) die zweite Abdeckung (71) erfasst, die von der Beleuchtungsvorrichtung (4) gelöst ist, und der Bildgebungsmodus auf den zweiten Bildgebungsmodus eingestellt ist.

17. Abbildungsvorrichtung (1) gemäß Anspruch 15, ferner aufweisend:
Einstellmittel (11) zum selektiven Einstellen, in Übereinstimmung mit einer von einem Benutzer der Abbildungsvorrichtung (1) ausgeführten Bedienung, einen Bildgebungsmodus zum Abbilden des Ziels mit dem Bildgeber (40) auf einen von mehreren Bildgebungsmodi, einschließlich eines ersten Bildgebungsmodus zum Abbilden des Ziels, das durch die mindestens eine erste Lichtquelle (141, 142, 143) beleuchtet wird, ohne die mindestens eine zweite Lichtquelle (144), und eines zweiten Bildgebungsmodus zum Abbilden des Ziels, das durch die mindestens eine zweite Lichtquelle (144) beleuchtet wird, ohne die mindestens eine erste Lichtquelle (141, 142, 143) zu beleuchten; und
Erfassungsmittel (62) zum Erfassen des Anbringens und Abnehmens der zweiten Abdeckung (71) an der bzw. von der Beleuchtungsvorrichtung (4),
wobei das Einstellmittel (11) den Abbildungsmodus in Reaktion darauf, dass das Erfassungsmittel (62) das Anbringen der zweiten Abdeckung (71) an der Beleuchtungsvorrichtung (4) in einem Zustand erfasst, in dem der Abbildungsmodus auf den ersten Abbildungsmodus eingestellt ist, in den zweiten Abbildungsmodus umschaltet und den Bildgebungsmodus auf einen der mehreren Bildgebungsmodi außer dem zweiten Bildgebungsmodus umschaltet, wenn die Erfassungseinrichtung (62) das Abnehmen der zweiten Abdeckung (71) von der Beleuchtungsvorrichtung (4) in einem Zustand erfasst, in dem der Bildgebungsmodus auf den zweiten Bildgebungsmodus eingestellt ist.

## Revendications

1. Dispositif d'imagerie (1) pour imager un corps humain comme cible, le dispositif d'imagerie (1) comprenant :
un dispositif d'illumination (4) approprié pour être utilisé avec un imageur (40) afin d'imager une cible, et l'imageur (40), dans lequel le dispositif d'illumination (4) comprend :
un couvercle d'objectif (72, 132) incluant un élément transmettant la lumière, le couvercle d'objectif (72, 132) étant configuré pour diriger la lumière depuis la cible vers l'imageur (40) afin de permettre à l'imageur (40) d'imager la cible ; et
au moins une source lumineuse (141, 142, 143, 144) située plus près de l'imageur (40) que le couvercle d'objectif (72, 132) pour illuminer la cible à travers le couvercle d'objectif (72, 132),
dans lequel l'imageur (40) inclut un capteur d'image (44) configuré pour imager la cible et une lentille d'imagerie (41a, 41b) configurée pour former une image de la cible sur le capteur d'image (44), et ladite au moins une source lumineuse (141, 142, 143, 144) est située à l'extérieur d'une région inversée, qui est une région formée en inversant substantiellement, vers l'imageur (40) et par rapport au couvercle d'objectif (72, 132), une région définie par des rayons prédéterminés de rayons imaginaires plus proches de la cible à partir du couvercle d'objectif (72, 132) dans un faisceau de rayons, le faisceau de rayons entrant, à travers la lentille d'imagerie (41a, 41b), dans le capteur d'image (44) en provenance de la cible en contact avec le couvercle d'objectif (72, 132) et en mise au point avec la lentille d'imagerie (41a, 41b),
dans lequel le couvercle d'objectif (72, 132) inclut un premier couvercle d'objectif (132) et un deuxième couvercle d'objectif (72), et au moins un couvercle parmi le premier couvercle d'objectif (132) et le deuxième couvercle d'objectif (72) est utilisable sélectivement,
le premier couvercle d'objectif (132) présente une première zone prédéterminée, qui est une zone d'une surface orthogonale à un axe optique de l'imageur (40),
le deuxième couvercle d'objectif (72) comporte une deuxième zone prédéterminée, qui est une zone d'une surface orthogonale à l'axe optique de l'imageur (40), et la deuxième zone est plus petite que la première zone,
ladite au moins une source lumineuse inclut au moins une première source lumineuse (141, 142, 143) pour illuminer la cible à travers le premier couvercle d'objectif (132) et au moins une deuxième source lumineuse (144) plus proche de l'axe optique pour illuminer la cible à travers le deuxième couvercle d'objectif (72),
ladite au moins une première source lumineuse (141, 142, 143) est située à l'extérieur d'une première région inversée, qui est une région formée en inversant substantiellement, vers l'imageur (40) et par rapport au premier couvercle d'objectif (132), une région définie par des premiers rayons prédéterminés de rayons imaginaires plus proches de la cible provenant du premier couvercle d'objectif (132) dans un faisceau de rayons, le faisceau de rayons entrant, à travers la lentille d'imagerie (41a, 41b), dans le capteur d'image (44) en provenance de la cible en contact avec le premier couvercle d'objectif (132) et en mise au point avec la lentille d'imagerie (41a, 41b), et
ladite au moins une deuxième source lumineuse (144) est située vers l'extérieur d'une deuxième région inversée, qui est une région formée en inversant substantiellement, vers l'imageur (40) et par rapport au deuxième couvercle d'objectif (72), une région définie par des deuxièmes rayons prédéterminés de rayons imaginaires plus proches de la cible à partir du deuxième couvercle d'objectif (72) dans un faisceau de rayons, le faisceau de rayons entrant, à travers la lentille d'imagerie (41a, 41b), dans le capteur d'image (44) en provenance de la cible en contact avec le deuxième couvercle d'objectif (72) et en mise au point avec la lentille d'imagerie (41a, 41b).

2. Dispositif d'imagerie (1) selon la revendication 1, dans lequel
parmi les rayons entrant dans le capteur d'image (44), les rayons prédéterminés incluent un rayon supérieur (171a, 175a) situé à une position la plus externe parmi une pluralité de rayons supérieurs inclus dans le faisceau de rayons, et un rayon inférieur (174a, 178a) situé à une position la plus externe parmi une pluralité de rayons inférieurs inclus dans le faisceau de rayons.

3. Dispositif d'imagerie (1) selon la revendication 1, dans lequel
parmi les rayons entrant dans le capteur d'image (44), les rayons prédéterminés incluent des rayons les plus externes (172a, 173a, 176a, 177a) situés aux positions les plus externes parmi une pluralité de rayons inclus dans le faisceau de rayons.

4. Dispositif d'imagerie (1) selon l'une quelconque des revendications 1 à 3, dans lequel
la région inversée est une région formée en inversant substantiellement la région définie par les rayons prédéterminés vers l'imageur (40) et par rapport à une surface du couvercle d'objectif (72, 132) adjacente à la cible.

5. Dispositif d'imagerie (1) selon l'une quelconque des revendications 1 à 4, dans lequel
ladite au moins une source lumineuse inclut une pluralité de sources lumineuses (141, 142, 143) agencées symétriquement les unes par rapport aux autres autour d'un axe optique de l'imageur (40) dans une direction orthogonale à l'axe optique de l'imageur (40).

6. Dispositif d'imagerie (1) selon la revendication 5, dans lequel
chaque source de la pluralité de sources lumineuses (141, 142, 143) est située pour émettre de la lumière dirigée vers le couvercle d'objectif (72, 132) pour se focaliser sur une zone globale du couvercle d'objectif (72, 132).

7. Dispositif d'imagerie (1) selon la revendication 5 ou 6, **caractérisé en ce que**
la pluralité de sources lumineuses (141, 142, 143) inclut au moins un type de source lumineuse sélectionné dans le groupe constitué par une source lumineuse de lumière visible, une source lumineuse de lumière ultraviolette, et une source lumineuse de lumière visible polarisée.

8. Dispositif d'imagerie (1) selon la revendication 1, dans lequel
les premiers rayons imaginaires incluent un rayon supérieur (171a) situé à une position la plus externe parmi une pluralité de rayons supérieurs inclus dans le faisceau de rayons, et un rayon inférieur (174a) situé à une position la plus externe parmi une pluralité de rayons inférieurs inclus dans le faisceau de rayons, le faisceau de rayons entrant, à travers la lentille d'imagerie (41a, 41b), dans le capteur d'image (44) en provenance de la cible en contact avec le premier couvercle d'objectif (132) et en mise au point avec la lentille d'imagerie (41a, 41b), et
les deuxièmes rayons imaginaires incluent un rayon supérieur (175a) situé à une position la plus externe parmi une pluralité de rayons supérieurs inclus dans le faisceau de rayons, et un rayon inférieur (178a) situé à une position la plus externe parmi une pluralité de rayons inférieurs inclus dans le faisceau de rayons, le faisceau de rayons entrant, à travers la lentille d'imagerie (41a, 41b), dans le capteur d'image (44) en provenance de la cible en contact avec le deuxième couvercle d'objectif (72) et en mise au point avec la lentille d'imagerie (41a, 41b).

9. Dispositif d'imagerie (1) selon la revendication 1, dans lequel
les premiers rayons prédéterminés incluent des rayons les plus externes (172a, 173a) situés aux positions les plus externes parmi une pluralité de rayons imaginaires inclus dans le faisceau de rayons, le faisceau de rayons entrant, à travers la lentille d'imagerie (41a, 41b), dans le capteur d'image (44) en provenance de la cible en contact avec le premier couvercle d'objectif (132) et en mise au point avec la lentille d'imagerie (41a, 41b), et
les deuxièmes rayons prédéterminés incluent des rayons les plus externes (176a, 177a) situés aux positions les plus externes parmi une pluralité de rayons imaginaires inclus dans le faisceau de rayons, le faisceau de rayons entrant, à travers la lentille d'imagerie (41a, 41b), dans le capteur d'image (44) en provenance de la cible en contact avec le deuxième couvercle d'objectif (72) et en mise au point avec la lentille d'imagerie (41a, 41b).

10. Dispositif d'imagerie (1) selon l'une quelconque des revendications 1 à 9, dans lequel
la première région inversée est une région formée en inversant substantiellement la région définie par les premiers rayons prédéterminés vers l'imageur (40) et par rapport à une surface du couvercle d'objectif (132) adjacente à la cible, et
la deuxième région inversée est une région formée en inversant substantiellement la région définie par les deuxièmes rayons prédéterminés vers l'imageur (40) et par rapport à la surface du couvercle d'objectif (72) adjacente à la cible.

11. Dispositif d'imagerie (1) selon l'une quelconque des revendications 1 à 10, dans lequel
ladite au moins une première source lumineuse (141, 142, 143) inclut une pluralité de premières sources lumineuses, situées chacune pour émettre de la lumière dirigée vers le premier couvercle d'objectif (132), et se focalise sur une zone globale du premier couvercle d'objectif (132), et
ladite au moins une deuxième source lumineuse (144) inclut une pluralité de deuxièmes sources lumineuses, situées chacune pour émettre de la lumière dirigée vers le deuxième couvercle d'objectif (72), et se focalise sur une zone globale du deuxième couvercle d'objectif (72).

12. Dispositif d'imagerie (1) selon la revendication 11, dans lequel
la pluralité de premières sources lumineuses et la pluralité de deuxièmes sources lumineuses incluent des sources lumineuses communes (151, 161), et
les sources lumineuses communes (151, 161) fonctionnent sélectivement comme la pluralité de premières sources lumineuses ou la pluralité de deuxièmes sources lumineuses en changeant les positions des sources lumineuses communes (151, 161) par rapport au premier couvercle d'objectif (132) ou au deuxième couvercle d'objectif (72), ou en changeant l'angle de la lumière émise par chacune des sources lumineuses communes (151, 161).

13. Dispositif d'imagerie (1) selon l'une quelconque des revendications 1 à 12, dans lequel
ladite au moins une première source lumineuse inclut au moins un type de source lumineuse sélectionné dans le groupe constitué par une source lumineuse de lumière visible, une source lumineuse de lumière ultraviolette et une source lumineuse de lumière visible polarisée.

14. Dispositif d'imagerie (1) selon l'une quelconque des revendications 1 à 13, dans lequel le couvercle d'objectif (72, 132) inclus dans le dispositif d'illumination (4) entre en contact avec la peau à imager avec le dispositif d'imagerie (1).

15. Dispositif d'imagerie (1) selon l'une quelconque des revendications 1 à 13, dans lequel le premier couvercle d'objectif (132) se trouve sur la partie supérieure d'un premier couvercle (131) inclus dans le dispositif d'illumination (4), et le premier couvercle (131) est cylindrique,
le deuxième couvercle d'objectif (72) est sur la partie supérieure d'un deuxième couvercle (71) et est cylindrique,
le deuxième couvercle (71) inclut, dans une portion autre que la partie supérieure, un moyen d'attachement et de détachement (71b) pour attacher et détacher le deuxième couvercle (71) sur le dispositif d'illumination (4),
le premier couvercle d'objectif (132) est utilisé comme couvercle d'objectif pour être en contact avec la peau, le deuxième couvercle (71) étant détaché du dispositif d'illumination (4) par le moyen d'attachement et de détachement (71b), et
le deuxième couvercle d'objectif (72) est utilisé comme couvercle d'objectif pour être en contact avec la peau, le deuxième couvercle (71) étant attaché au dispositif d'illumination (4) par le moyen d'attachement et de détachement (71b) et recouvrant le premier couvercle (131).

16. Dispositif d'imagerie (1) selon la revendication 15, comprenant en outre :
un moyen de réglage (11) pour régler sélectivement, conformément à une opération effectuée par un utilisateur du dispositif d'imagerie (1), un mode d'imagerie pour imager la cible avec l'imageur (40) dans un mode parmi une pluralité de modes d'imagerie incluant un premier mode d'imagerie pour imager la cible illuminée par ladite au moins une première source lumineuse (141, 142, 143) sans ladite au moins une deuxième source lumineuse (144) et un deuxième mode d'imagerie pour imager la cible illuminée par ladite au moins une deuxième source lumineuse (144) sans ladite au moins une première source lumineuse (141, 142, 143) ;
un moyen de détection (62) pour détecter le deuxième couvercle (71) dans un état attaché ou détaché sur le dispositif d'illumination (4) ; et
un moyen d'indication (11) pour informer, en réponse à ce qu'au moins une des conditions est satisfaite, l'utilisateur du fait que ladite au moins une condition est satisfaite, les conditions incluant la détection, par le moyen de détection (62), du deuxième couvercle (71) attaché au dispositif d'illumination (4) et le réglage du mode d'imagerie sur le premier mode d'imagerie, et la détection, par le moyen de détection (62), du deuxième couvercle (71) détaché du dispositif d'illumination (4) et le réglage du mode d'imagerie sur le deuxième mode d'imagerie.

17. Dispositif d'imagerie (1) selon la revendication 15, comprenant en outre :
un moyen de réglage (11) pour régler sélectivement, conformément à une opération effectuée par un utilisateur du dispositif d'imagerie (1), un mode d'imagerie pour imager la cible avec l'imageur (40) dans un mode parmi une pluralité de modes d'imagerie incluant un premier mode d'imagerie pour imager la cible illuminée par ladite au moins une première source lumineuse (141, 142, 143) sans ladite au moins une deuxième source lumineuse (144) et un deuxième mode d'imagerie pour imager la cible illuminée par ladite au moins une deuxième source lumineuse (144) sans ladite au moins une première source lumineuse (141, 142, 143) ; et
un moyen de détection (62) pour détecter l'attachement et le détachement du deuxième couvercle (71) sur le dispositif d'illumination (4),
dans lequel le moyen de réglage (11) commute le mode d'imagerie au deuxième mode d'imagerie en réponse à une détection, par le moyen de détection (62), de l'attachement du deuxième couvercle (71) sur le dispositif d'illumination (4) dans un état où le mode d'imagerie est réglé sur le premier mode d'imagerie, et commute le mode d'imagerie à un mode de la pluralité de modes d'imagerie autre que le deuxième mode d'imagerie en réponse à une détection, par le moyen de détection (62), du détachement du deuxième couvercle (71) du dispositif d'illumination (4) dans un état où le mode d'imagerie est réglé sur le deuxième mode d'imagerie.
